# EUROPEAN PATENT SPECIFICATION

(11) **EP 2 263 514 B1**
(45) Date of publication and mention of the grant of the patent: **10.10.2018**
(21) Application number: 10009960.5
(22) Date of filing: 28.08.2007
(51) Int. Cl.: A61B 1/005, A61B 1/018, A61B 8/12

(54) **Ultrasonic endoscope**
Ultraschallendoskop
Endoscope ultrasonique

(30) Priority: 28.08.2006 US 823669 P
(43) Date of publication of application: 22.12.2010
(62) Divisional of application: 07016829.9
(73) Proprietor: Olympus Corporation, Tokyo 192-8507 (JP)
(72) Inventor: Sato, Masatoshi, Hachioji-shi Tokyo 192-8507 (JP); Kaji, Kunihide, Hachioji-shi Tokyo 192-8507 (JP); Suzuki, Takayuki, Hachioji-shi Tokyo 192-8507 (JP); Shiono, Junji, Hachioji-shi Tokyo 192-8507 (JP); Mikkaichi, Takayasu, Hachioji-shi Tokyo 192-8507 (JP); Shinichi, Tsutaki, Hachioji-shi Tokyo 192-8507 (JP); Toshihiro, Shizuka, Hachioji-shi Tokyo 192-8507 (JP); Takeharu, Nakazato, Hachioji-shi Tokyo 192-8507 (JP); Sato, Sunao, Hachioji-shi Tokyo 192-8507 (JP); Mizunuma, Akiko, Hachioji-shi Tokyo 192-8507 (JP)
(74) Representative: Gunzelmann, Rainer

(56) References cited:
- EP-A- 0 269 791
- JP-A- 7 116 166
- JP-A- 2001 292 996
- US-A1- 2004 082 883
- US-A1- 2006 009 681

## Description

The present invention relates to an ultrasonic endoscope.

Jpn. Pat. Appln. KOKAI Publication No. 2001-292997 discloses an ultrasonic endoscope. This ultrasonic endoscope includes an insertion section and an operation section disposed at the proximal end of the insertion section. An objective lens for optical observation is fixed to the distal end of the insertion section. An ultrasonic transducer is capable of projecting from the distal end of the insertion section. This allows ultrasonic observation, even with the ultrasonic transducer projecting from the distal end of the insertion section.

The ultrasonic endoscope of Jpn. Pat. Appln. KOKAI Publication No. 2001-292997 has problems in that the focal distance required for optical observation may not be ensured depending upon how the ultrasonic transducer projects from the distal end of the insertion section.

In addition, there may be a case where one person has to operate two or more endoscopes or therapeutic tools, as in the case where an endoscope for optical observation and an endoscope for ultrasonic observation are used independently. In such a case, it is desirable to use an instrument that permits the endoscopes or therapeutic tools to be held together.

There is a case where a T-bar is used for pulling the living tissue. If the T-bar cannot be optically observed, its position is recognized by ultrasonic observation, for example. However, the T-bar is not easily recognizable by ultrasonic observation, there is a demand for technology that enables easy recognition of the T-bar based on ultrasonic observation. There is also a demand for a tool that guides the T-bar to a position at which the T-bar cannot be optically observed.

The present invention has been made in an effort to solve the problems described above, and the first object of the invention is to provide an ultrasonic endoscope which provides improved insertion efficiency and enables simultaneous execution of both optical observation and ultrasonic observation.

According to one aspect of the present invention, there is provided an ultrasonic endoscope including the features of claim 1.

The invention can be more fully understood from the following detailed description when taken in conjunction with the accompanying drawings, in which:
FIG. 1 is a schematic view of an ultrasonic endoscope according to a first embodiment of the present invention;
FIG. 2 is a schematic vertical cross-sectional view showing the distal end of the insertion section of the ultrasonic endoscope according to the first embodiment;
FIG. 3A is a schematic view of the distal end of the insertion section of the ultrasonic endoscope according to the first embodiment, in which the central axis (scanning surface) of an ultrasonic transducer (an
   ultrasonic oscillator) incorporated in an ultrasonic probe is on the central axis of a first working channel (a first forceps channel);
FIG. 3B is a schematic view of the distal end of the insertion section of the ultrasonic endoscope according to the first embodiment, in which the central axis of the ultrasonic transducer incorporated in the ultrasonic probe is on the central axis of a second working channel (a second forceps channel);
FIG. 4A is a schematic view showing the insertion section of the ultrasonic endoscope according to the first embodiment and illustrating the operating state of the ultrasonic probe inserted in an operation section;
FIG. 4B is a schematic view showing the operation section of the ultrasonic endoscope according to the first embodiment and illustrating the state in which an ultrasonic transducer cable is pushed out toward the distal end of the insertion section by moving a slider away from a pivotal support part of a movable lever;
FIG. 4C is a schematic view showing the operation section of the ultrasonic endoscope according to the first embodiment and illustrating the state in which the ultrasonic transducer cable is pulled toward the proximal end of the insertion section by moving the slider closer to the pivotal support part of the movable lever;
FIG. 5A is a schematic vertical cross-sectional view taken along the line 5A-5A of FIG. 5B, showing the vicinity of a rotation knob in FIG. 4A disposed in the insertion section of the ultrasonic endoscope according to the first embodiment;
FIG. 5B is a schematic vertical cross-sectional view taken along the line 5B-5B of FIG. 5A, showing the vicinity of the rotation knob disposed in the insertion section of the ultrasonic endoscope according to the first embodiment;
FIG. 5C is a schematic transverse sectional view taken along the line 5C-5C of FIG. 5A, showing the rotation knob disposed in the insertion section of the ultrasonic endoscope according to the first embodiment;
FIG. 6 is a schematic block diagram of an endoscopic system according to the first embodiment, which incorporates an ultrasonic endoscope, video processor, and ultrasonic observation device;
FIG. 7A is a schematic view showing an optical observation monitor coupled to the video processor of the endoscopic system according to the first embodiment, and illustrating the state in which an optically observed image is shown on a display screen of the monitor and a grid is superimposed on the optically observed image;
FIG. 7B is a schematic view showing the optical observation monitor coupled to the video processor of the endoscopic system according to the first embodiment, and illustrating the state in which an optically observed image is shown on the display screen of the monitor and a scale is superimposed on the optically observed image;
FIG. 8A is a schematic perspective view illustrating the state in which the ultrasonic transducer of the ultrasonic probe and a holding portion for the ultrasonic transducer project from the distal end of the insertion section of the ultrasonic endoscope according to the first embodiment, and the scanning surface of the ultrasonic transducer is disposed on the central axis of the second working channel;
FIG. 8B is a schematic perspective view illustrating the state in which the ultrasonic transducer, holding portion, and semispherical part of the ultrasonic probe project from the distal end of the insertion section of the ultrasonic endoscope according to the first embodiment, and the holding portion is rotated so that the scanning surface of the ultrasonic transducer is disposed on the central axis of the first working channel;
FIG. 8C is a schematic perspective view illustrating the state in which the ultrasonic transducer, holding portion, and semispherical part of the ultrasonic probe projecting from the distal end of the insertion section of the ultrasonic endoscope according to the first embodiment are pulled to decrease the degree of projection, and the scanning surface of the ultrasonic transducer is disposed on the central axis of the first working channel;
FIG. 9 is a schematic block diagram showing a modified example of the endoscopic system according to the first embodiment, which incorporates the ultrasonic endoscope, video processor, and ultrasonic observation device;
FIG. 10 is a schematic block diagram showing another modified example of the endoscopic system according to the first embodiment, which incorporates the ultrasonic endoscope, video processor, and ultrasonic observation device;
FIG. 11A is a schematic view of the distal end of the insertion section of an ultrasonic endoscope according to a second embodiment;
FIG. 11B is a schematic view of the distal end of an ultrasonic probe of the ultrasonic endoscope according to the second embodiment;
FIG. 12A is a schematic perspective view showing the distal end of the insertion section of an ultrasonic endoscope according to a third embodiment and illustrating the state in which an ultrasonic transducer of an ultrasonic probe projects from the face of the distal end of the insertion section;
FIG. 12B is a schematic vertical sectional view showing the distal end of the insertion section of the ultrasonic endoscope according to the third embodiment and illustrating the state in which the ultrasonic transducer of the ultrasonic probe projects from the face of the distal end of the insertion section;
FIG. 12C is a schematic perspective view showing the distal end of the insertion section of the ultrasonic endoscope according to the third embodiment and illustrating the state in which the ultrasonic transducer of the ultrasonic probe is disposed in substantially the same plane as the face of the distal end of the insertion section;
FIG. 12D is a schematic vertical sectional view showing the distal end of the insertion section of the ultrasonic endoscope according to the third embodiment and illustrating the state in which the ultrasonic transducer of the ultrasonic probe is disposed in substantially the same plane as the face of the distal end of the insertion section;
FIG. 13 is a schematic perspective view showing the distal end of the insertion section of an ultrasonic endoscope according to a fourth embodiment and illustrating the state in which an ultrasonic transducer of an ultrasonic probe projects from the face of the distal end of the insertion section;
FIG. 14A is a schematic view of the distal end of the insertion section of an ultrasonic endoscope according to a fifth embodiment;
FIG. 14B is a schematic vertical sectional view taken along the line 14B-14B of FIG. 14A, showing the distal end of the insertion section of the ultrasonic endoscope according to the fifth embodiment;
FIG. 14C is a schematic vertical sectional view showing the distal end of the insertion section of the ultrasonic endoscope according to the fifth embodiment and illustrating the state in which a surgical instrument is inserted in a working channel shown in FIG. 14B;
FIG. 15A is a schematic view illustrating the state in which the surgical instrument passed through the working channel intersects the ultrasonic oscillating surface of the ultrasonic endoscope according to the fifth embodiment;
FIG. 15B is a schematic view illustrating the state in which the surgical instrument passed though the working channel is parallel to the ultrasonic oscillating surface of the ultrasonic endoscope according to the fifth embodiment;
FIG. 16A is a schematic front view of the distal end of the insertion section of an ultrasonic endoscope according to a sixth embodiment;
FIG. 16B is a schematic vertical sectional view of the distal end of the insertion section of the ultrasonic endoscope according to the sixth embodiment;
FIG. 17 is a schematic vertical sectional view of the distal end of the insertion section of an ultrasonic endoscope according to a seventh embodiment;
FIG. 18 is a schematic vertical sectional view of the distal end of the insertion section of an ultrasonic endoscope according to an eighth embodiment;
FIG. 19 is a schematic view illustrating the state in which one of two ultrasonic endoscopes according to a ninth embodiment is introduced in a stomach through the mouth and the other is introduced to a site outside the stomach through the skin;
FIG. 20 is a schematic view illustrating the state in which an ultrasonic wave is transmitted and received between the distal ends of the insertion sections of the two ultrasonic endoscopes according to the ninth embodiment in order to search for the opposite positions of the distal ends;
FIG. 21A is a schematic view illustrating the state in which one of the two ultrasonic endoscopes according to the ninth embodiment is placed in an intestine through the mouth or anus;
FIG. 21B is a schematic view illustrating the state in which one of the two ultrasonic endoscopes according to the ninth embodiment is placed in an intestine through the mouth or anus, and the other is introduced by way of the skin in order to push away an adjacent organ such that an ultrasonic wave is transmitted to or received from the distal end of the insertion section of the one ultrasonic endoscope to search for the opposite positions;
FIG. 21C is a schematic view illustrating the state in which the curvbable part of the other one of the two ultrasonic endoscopes according to the ninth embodiment is operated, thereby defining a space between the intestinal wall and the organ while holding the opposite positions of the distal ends of the insertion sections of the two ultrasonic endoscopes;
FIG. 21D is a schematic view illustrating the state in which a needle from one of the two ultrasonic endoscopes according to the ninth embodiment pierces through the intestinal wall that includes an area of interest, and the distal end of the insertion section of the other ultrasonic endoscope is disposed beyond the needle point;
FIG. 22A is a schematic perspective view of an endoscopic system according to a tenth embodiment;
FIG. 22B is a schematic view showing an optical observation endoscope, an ultrasonic observation endoscope, and an ultrasonic probe, all of which project from the distal end of the sheath of the endoscopic system according to the tenth embodiment;
FIG. 22C is a schematic view of the distal end of the part (represented by reference number 22C in FIG. 22B) of the insertion section of the optical observation endoscope of the endoscopic system according to the tenth embodiment;
FIG. 22D is a schematic view of the distal end of the part (represented by reference number 22D in FIG. 22B) of the insertion section of the ultrasonic observation endoscope of the endoscopic system according to the tenth embodiment;
FIG. 22E is a schematic view of the distal end of the part (represented by reference number 22E in FIG. 22B) of the insertion section of the ultrasonic probe of the endoscopic system according to the tenth embodiment;
FIG. 23 is a schematic view for explaining that the optical observation endoscope can be disposed in a slot of the main body case of the endoscopic system according to the tenth embodiment;
FIG. 24 is a schematic view of the back of the main body case of the endoscopic system according to the tenth embodiment;
FIG. 25A is a schematic view illustrating the state in which while a body wall is observed using the optical observation endoscope of the endoscopic system according to the tenth embodiment, a needle is projected from a channel and pierced through a site away from an area of interest, and the area of interest is ultrasonically observed using the ultrasonic observation endoscope;
FIG. 25B is a schematic view illustrating the state in which while a body wall and an area of interest are observed using the optical observation endoscope and the ultrasonic observation endoscope, respectively, of the endoscopic system according to the tenth embodiment, the ultrasonic probe is introduced from the site pierced with the needle, and the ultrasonic transducer at the distal end of the ultrasonic probe is disposed behind the area of interest;
FIG. 25C is a schematic view illustrating the state in which while a body wall and an area of interest are observed using the optical observation endoscope and the ultrasonic observation endoscope, respectively, of the endoscopic system according to the tenth embodiment, an organ is pushed away from the body wall using the distal end of the ultrasonic probe, thereby defining a space;
FIG. 26 is the schematic front view of the distal end of the insertion section of an ultrasonic endoscope according to an eleventh embodiment;
FIG. 27A is a schematic vertical sectional view illustrating the state in which the distal end of a cap attached to the distal end of the insertion section of the ultrasonic endoscope according to the eleventh embodiment is in contact with a body wall;
FIG. 27B is a schematic vertical sectional view illustrating the state in which under ultrasonic observation, the living tissue is suctioned into the cap attached to the distal end of the insertion section of the ultrasonic endoscope according to the eleventh embodiment;
FIG. 27C is a schematic vertical sectional view illustrating the state in which under ultrasonic observation, the living tissue is suctioned into the cap attached to the distal end of the insertion section of the ultrasonic endoscope according to the eleventh embodiment, and a needle is pierced through a body wall via a suction passage (a suction channel);
FIG. 27D is a schematic vertical sectional view illustrating the state in which under ultrasonic observation, the living tissue is suctioned into the cap attached to the distal end of the insertion section of the ultrasonic endoscope according to the eleventh embodiment, a needle having a T-bar is pierced through the body wall via the suction passage, and the T-bar is placed on the body wall;
FIG. 28A is the schematic front view of the distal end of the insertion section of an ultrasonic endoscope according to a twelfth embodiment;
FIG. 28B is a schematic vertical sectional view illustrating the state in which the distal end face of the insertion section of the ultrasonic endoscope according to the twelfth embodiment is brought into contact with a body wall;
FIG. 28C is a schematic vertical sectional view illustrating the state in which a needle is pierced through a body wall while the face of the distal end of the insertion section of the ultrasonic endoscope according to the twelfth embodiment is in contact with the body wall;
FIG. 29 is a schematic view of a T-bar suturing device according to a thirteenth embodiment;
FIG. 30A is a schematic perspective view of a T-bar placed using the T-bar suturing device according to the thirteenth embodiment;
FIG. 30B is a schematic vertical sectional view of the T-bar placed using the T-bar suturing device according to the thirteenth embodiment;
FIG. 31A is a schematic perspective view of an example of the shape of the T-bar placed using the T-bar suturing device according to the thirteenth embodiment;
FIG. 31B is a schematic perspective view of another example of the shape of the T-bar placed using the T-bar suturing device according to the thirteenth embodiment;
FIG. 31C is a schematic perspective view of another example of the shape of the T-bar placed using the T-bar suturing device according to the thirteenth embodiment;
FIG. 32 is a schematic view of a needle tube of the T-bar suturing device according to the thirteenth embodiment and a bar of the T-bar;
FIG. 33 is a schematic perspective view of the needle tube of the T-bar suturing device according to the thirteenth embodiment;
FIG. 34A is a schematic perspective view illustrating the state in which the bar of the T-bar is about to be loaded into the needle tube of the T-bar suturing device according to the thirteenth embodiment;
FIG. 34B is a schematic perspective view illustrating the state in which the bar of the T-bar is loaded in the needle tube of the T-bar suturing device according to the thirteenth embodiment;
FIG. 34C is a schematic perspective view illustrating the state in which the bar of the T-bar loaded in the needle tube of the T-bar suturing device according to the thirteenth embodiment is covered with a sheath;
FIG. 35 is a schematic perspective view illustrating the state in which the sheath covering the bar of the T-bar loaded in the needle tube of the T-bar suturing device according to the thirteenth embodiment is moved away toward the proximal end side;
FIG. 36A is a schematic view illustrating the state in which a body wall is pierced with the distal end of the needle tube of the T-bar suturing device according to the thirteenth embodiment;
FIG. 36B is a schematic view illustrating the state in which the body wall is pierced with the distal end of the needle tube of the T-bar suturing device according to the thirteenth embodiment, and the bar of the T-bar is caused to fall with a pusher while the distal end of the needle tube is observed using an ultrasonic endoscope;
FIG. 36C is a schematic view illustrating the state in which the bar of the T-bar is caused to fall from the distal end of the needle tube of the T-bar suturing device according to the thirteenth embodiment, and then the needle tube of the T-bar suturing device is pulled off the body wall;
FIG. 36D is a schematic view illustrating the state in which after the T-bar is placed on a body wall using the T-bar suturing device according to the thirteenth embodiment, a thread member or spherical member of the T-bar is grasped using another grasping forceps;
FIG. 36E is a schematic view illustrating the state in which after the T-bar is placed on the body wall using the T-bar suturing device according to the thirteenth embodiment and the thread member or spherical member of the T-bar is grasped using the another grasping forceps, a stopper is moved closer to the bar along the thread member using the distal end of the sheath covering the periphery of the grasping forceps;
FIG. 37 is a schematic perspective view of a T-bar having a double bar, which is placed using the T-bar suturing device according to the thirteenth embodiment;
FIG. 38 is a schematic perspective view of another T-bar having a double bar, which is placed using the T-bar suturing device according to the thirteenth embodiment;
FIG. 39 is a schematic vertical sectional view illustrating the state in which a T-bar having a double bar is disposed in the needle tube of the T-bar suturing device according to the thirteenth embodiment;
FIG. 40 is a schematic view of a sheath inserted in the working channel of an ultrasonic endoscope according to a fourteenth embodiment;
FIG. 41A is a schematic vertical sectional view of a tip (a distal end) of the sheath inserted in the working channel of the ultrasonic endoscope according to the fourteenth embodiment;
FIG. 41B is a schematic vertical sectional view illustrating the state in which a balloon at the tip of the sheath inserted in the working channel of the ultrasonic endoscope according to the fourteenth embodiment is inflated;
FIG. 42 is a schematic vertical sectional view of the proximal end of the sheath inserted in the working channel of the ultrasonic endoscope according to the fourteenth embodiment;
FIG. 43 is a schematic vertical sectional view of the balloon disposed at the tip of the sheath inserted in the working channel of the ultrasonic endoscope according to the fourteenth embodiment;
FIG. 44A is a schematic vertical sectional view illustrating ultrasonic observation with no sheath inserted in the working channel of the ultrasonic endoscope according to the fourteenth embodiment; and
FIG. 44B is a schematic vertical sectional view illustrating ultrasonic observation with the balloon inflated from the sheath inserted in the working channel of the ultrasonic endoscope according to the fourteenth embodiment.

Referring to the accompanying drawings, there will be described presently preferred embodiments of the invention. The ninth to fourteenth embodiments are examples which are not part of the present invention.

### (First Embodiment)

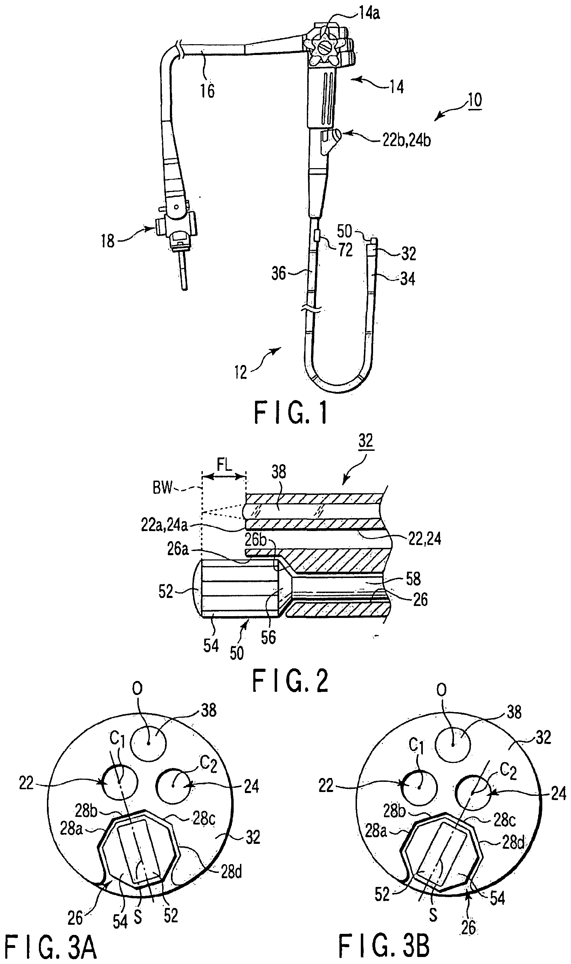

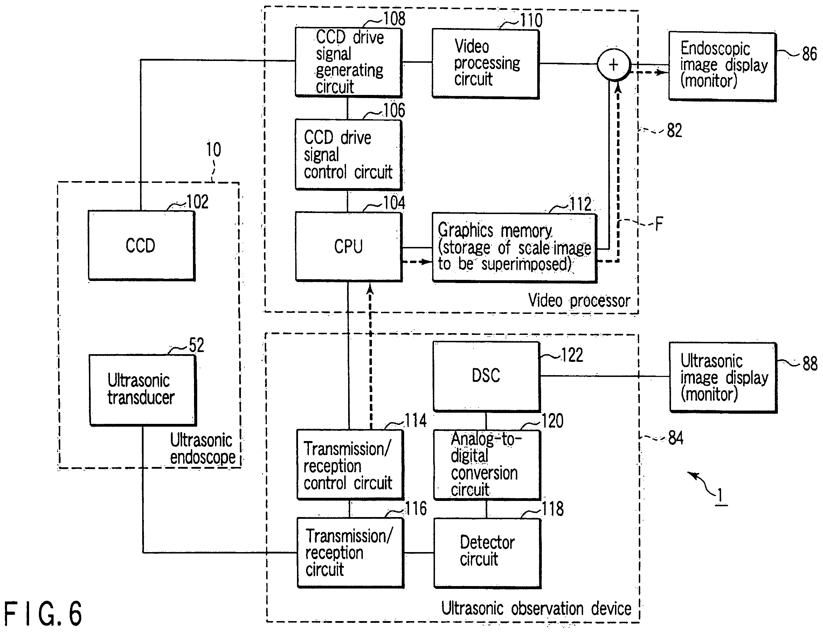

A description of a first embodiment will be given with reference to FIGS. 1 to 10. An ultrasonic endoscope 10 shown in FIG. 1 has an ultrasonic observation function for ultrasonically observing a subject using an ultrasonic wave, and an optical observation function for optically observing the subject. The ultrasonic endoscope 10 includes a long, thin insertion section 12, an operation section 14 attached to the proximal end of the insertion section 12, and a universal cord 16 extending from the operation section 14. Mounted on the universal cord 16 are a light source (not shown), an ultrasonic observation device 84 shown in FIG. 6, and a connector 18 to which a video processor 82 is connected.

As shown in FIGS. 2 to 3B, inserted in a part extending from the insertion section 12 to the operation section 14 are a first working channel (surgical instrument insertion channel, forceps channel) 22, a second working channel (surgical instrument insertion channel, forceps channel) 24, and an ultrasonic probe channel 26, all of which are parallel to one another.

As shown in FIG. 1, the insertion section 12 has a distal end rigid portion 32, a bending portion 34, and a flexible tube 36. The flexible tube 36 is flexible according to a reaction force exerted from the wall of a body cavity inside a body cavity when the insertion section 12 is inserted into such a passage. The bending portion 34 can be curved in a desired direction by rotating a bending-operation knob 14a of the operation section 14. As shown in FIGS. 3A and 3B, the distal end rigid portion 32 includes an optical observation objective lens (an optical observation system) 38, working channel openings 22a and 24a for the working channels 22 and 24, and a probe channel opening 26a for the ultrasonic probe channel 26. The distal end rigid portion 32 has an illuminating lens (not shown), which emits an illumination light to illuminate a subject being optically observed.

As shown in FIGS. 3A and 3B, the probe channel opening 26a of the probe channel 26 has an approximately regular hexagonal shape. In the probe channel opening 26a, part of periphery is removed in order to narrow the outer diameter of the distal end rigid portion 32. While the distal end surface of the distal end rigid portion 32 of the probe channel opening 26a is an approximately regular hexagon, the proximal end portion of the insertion section 12 has an approximately semicircular projecting edge 26b. Extending from the proximal end of the semicircular edge 26b is a flexible tube 26c the inside diameter of which is smaller than the diameter of the channel opening 26a with which the tube 26c is in contact. Thus, the channel opening 26a of the distal end rigid portion 32, the semicircular edge 26b and the flexible tube 26c form the ultrasonic probe channel 26.

The first channel opening 22a defined in the distal end face of the insertion section 12 of the endoscope 10 is disposed such that out of the four edges 28a, 28b, 28c, and 28d of the regular hexagonal shape of the probe channel 26 in which an ultrasonic probe 50 (described below) is disposed, the first channel opening 22a is perpendicular to the second edge 28b and passes through the center of the regular hexagon. The second channel opening 24a is perpendicular to the third edge 28c and also passes through the center of the regular hexagon. Additionally, the objective lens 38 is disposed in such a position that a surgical instrument or the like (not shown) can be viewed from the working channel openings 22a and 24a of the first channel 22 and second channel 24, respectively. It is preferable that the distance from the objective lens 38 to the center C₁ of the working channel opening 22a of the first channel 22 and the distance from the objective lens 38 to the center C₂ of the working channel opening 24a of the second channel 24 be equal. That is, an equilateral triangle or isosceles triangle is formed having, as its vertices, the center O of the objective lens 38, the center C₁ of the first working channel opening 22a, and the center C₂ of the second working channel opening 24a.

The proximal ends of the first and second working channels 22 and 24 are attached to the operation section 14. Adjacent to the forceps openings of the proximal end sides of the working channels 22 and 24, for example, are forceps valves 22b and 24b.

A surgical instrument or a forceps are insertable into the first and second working channels 22 and 24. Thus, the surgical instrument or the forceps can insert into the body cavity. Liquid or gas are able to be fed into/suctioned from the body cavity through the first and second working channels 22 and/or 24.

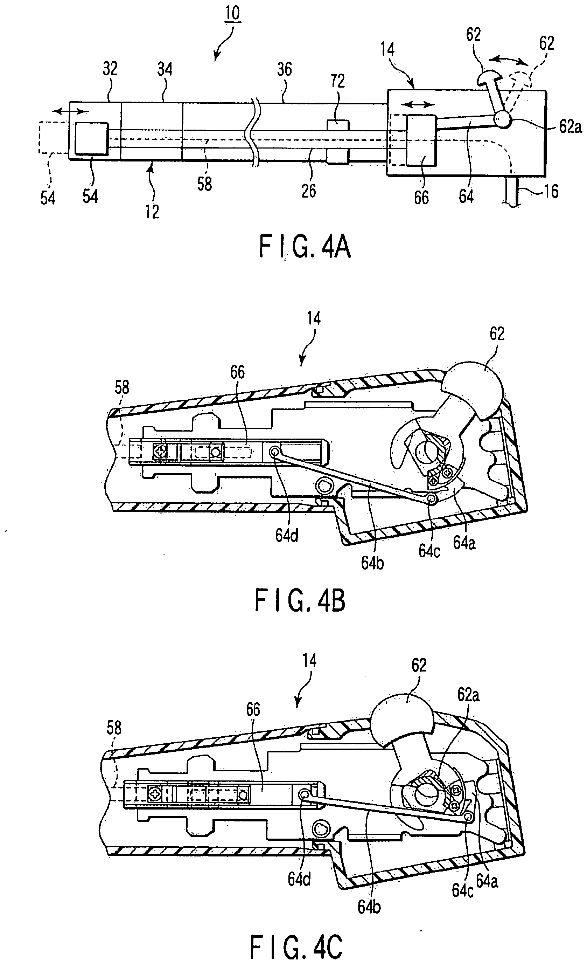

Referring to FIG. 4A, there is shown the ultrasonic probe channel 26 in which a long thin ultrasonic probe (ultrasonic observation system) 50 is disposed. As shown in FIG. 2, the ultrasonic probe 50 includes: an electronic convex type ultrasonic transducer 52 for ultrasonic observation; a holding portion 54 of approximately regular hexagonal shape, which holds the ultrasonic transducer 52 at its distal end; a semispherical part 56 disposed at the proximal end of the holding portion 54; and a flexible transducer cable 58 disposed at the proximal end of the semispherical part 56. The transducer cable 58, semispherical part 56, holding portion 54, and ultrasonic transducer 52 are disposed on the same axis. The descriptions given below exemplify the case where the holding portion 54 has the shape of an approximately regular hexagonal prism. However, any other regular polygonal prism, such as a regular pentagonal prism, is also suitable. According to the shape of the holding portion 54, the shape of the ultrasonic probe channel 26 also changes. Further, it is also preferable that the holding portion 54 have the shape of an approximately regular polygonal pyramid, such as an approximately regular hexagonal pyramid. According to the shape of the holding portion 54, the shape of the ultrasonic probe channel 26 also changes. In this case, the large cross-sectional area side of the holding portion 54 sticks out beyond the distal end face of the insertion section 12 whereas the small cross-sectional area side of the holding portion 54 is located on the proximal end side of the insertion section 12 rather than on the distal end face side thereof.

The transducer cable 58 is inserted in the ultrasonic probe channel 26. The semispherical part 56 is fitted in the approximately semispherical portion of the proximal end of the probe channel opening 26a. The holding portion 54 is formed in such a size as to fit in the probe channel opening 26a of the ultrasonic probe channel 26. The ultrasonic transducer 52 is disposed on the central axis of the approximately regular hexagonal holding portion 54. Accordingly, the holding portion 54 of the distal end of the ultrasonic probe 50, when normally used, is prevented from rotating relative to the probe channel opening 26a of the ultrasonic probe channel 26. As shown in FIGS. 3A and 3B, a scanning surface (oscillation surface) S of the oscillator 52 is selectively directed toward the first working channel 22 or second working channel 24.

As shown in FIG. 2, the axial distance from the distal end of the holding portion 54 to the semispherical part 56 is greater than the distance from the probe channel opening 26a of the ultrasonic probe channel 26 to the semispherical edge 26b. Accordingly, the holding portion 54 sticks out beyond the probe channel opening 26a. To be more specific, the ultrasonic transducer 52 projects from the distal end face of the distal end rigid portion 32 of the insertion section 12 of the endoscope 10.

The periphery of the transducer cable 58 has a recess 58a in which a drive pin 74 (described below) is disposed.

Referring to FIGS. 4B and 4C, there is shown the operation section 14, on which an axially movable lever 62 is pivotally supported by a pivotal support part 62a in order to move the ultrasonic probe 50 in the axial direction of the insertion section 12. The movable lever 62 is connected to the proximal end of the transducer cable 58 of the ultrasonic probe 50 via a link 64 and a slider 66.

By operating the movable lever 62 of the operation section 14, the slider 66 moves in the axial direction of the insertion section 12 (i.e., the axial direction of the ultrasonic probe channel 26) via the link 64. Consequently, the transducer cable 58, the proximal end of which is connected to the slider 66, moves in the axial direction of the insertion section 12. As a result, the semispherical part 56 and holding portion 54 disposed at the distal end of the transducer cable 58 move in the axial direction of the insertion section 12.

The link 64 includes a first link member 64a, a second link member 64b, a first pivotal support part 64c, and a second pivotal support part 64d. One end of the first link member 64a is pivotally supported by the pivotal support part 62a of the movable lever 62. The other end of the first link member 64a is connected to one end of the second link member 64b via the first pivotal support part 64c. The other end of the second link member 64b is connected to the slider 66 via the second pivotal support part 64d.

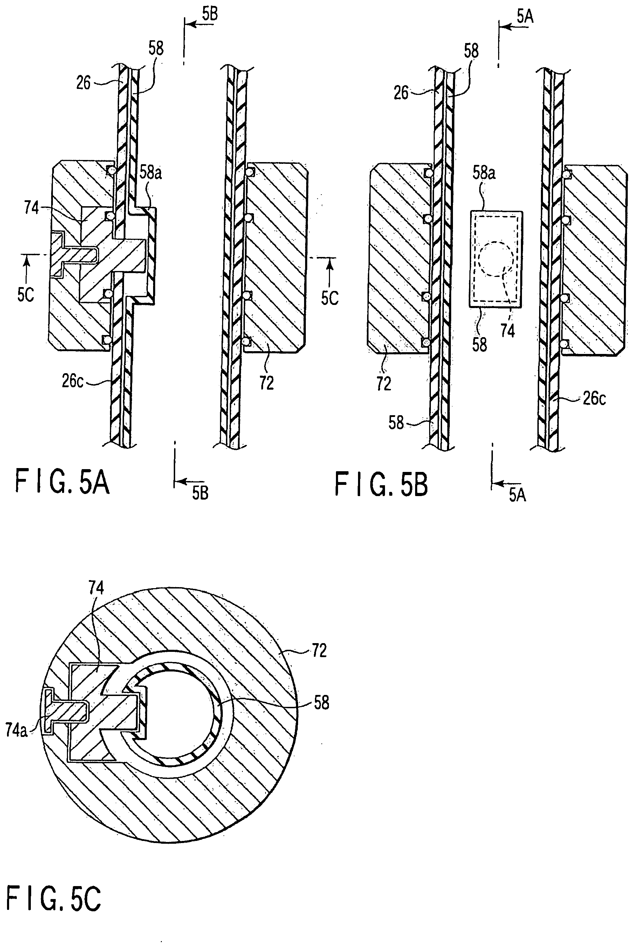

As shown in FIGS. 4A and 5A to 5C, at the proximal end of the insertion section 12 is a rotation knob 72 disposed via a plurality of O-rings such that at least part of the rotational knob 72 is exposed from the periphery of the insertion section 12 and the rotation knob 72 is rotatable around the axis of the probe channel 26. The drive pin 74 is fixed to the rotation knob 72 by a lock pin 74a from outside the knob 72. The distal end of the drive pin 74 extends, through the periphery of the probe channel 26, into the recess 58a formed in part of the transducer cable 58. Accordingly, by rotating the rotation knob 72 around the axis of the insertion section 12, the force is transmitted to the transducer cable 58 via the drive pin 74. Consequently, the transducer cable 58 rotates around the axis of the probe channel 26. As a result, the holding portion 54 disposed at the distal end of the transducer cable 58 also rotates around the axis of the probe channel 26.

As shown in FIG. 2, in the state where the holding portion 54 of the ultrasonic probe 50 is fixed to the channel opening 26a of the probe channel 26, a focal length FL of the objective lens 38 of the observing optical system is approximately equal to the distance from the distal end face of the insertion section 12 of the endoscope 10 to the ultrasonic transducer 52 of the ultrasonic probe 50. This makes it possible to observe substantially the same living tissue in a living body both optically and ultrasonically.

In this embodiment, as shown in FIGS. 5A and 5C, the recess 58a is formed in the transducer cable 58 and the drive pin 74 is disposed in the recess 58a. However, the invention is not limited to such a configuration. For example, it may be preferable that a projection be formed on the periphery of the transducer cable 58 and gripped by a gripper disposed on the rotation knob 72. The rotation of the rotation knob 72 in such a configuration is transmitted to the projection of the transducer cable 58 gripped by the gripper and consequently the transducer cable 58 rotates. In this case, eliminating the need to form the recess 58a in the transducer cable 58 prevents the internal configuration of the transducer cable 58 from being affected. In addition, since the rotation knob 72 is disposed at the proximal end of the insertion section 12, this does not affect the diameter of the insertion section 12, which is inserted in a passage in a body cavity.

As shown in FIG. 6, the endoscopic system 1 includes an optical observation monitor 86 and an ultrasonic observation monitor 88 in addition to the ultrasonic endoscope 10, the video processor 82, and the ultrasonic observation device 84. The ultrasonic endoscope 10 is electrically coupled to the video processor 82 and ultrasonic observation device 84. The video processor 82 is electrically coupled to the optical observation monitor 86, and the ultrasonic observation device 84 is electrically coupled to the ultrasonic observation monitor 88.

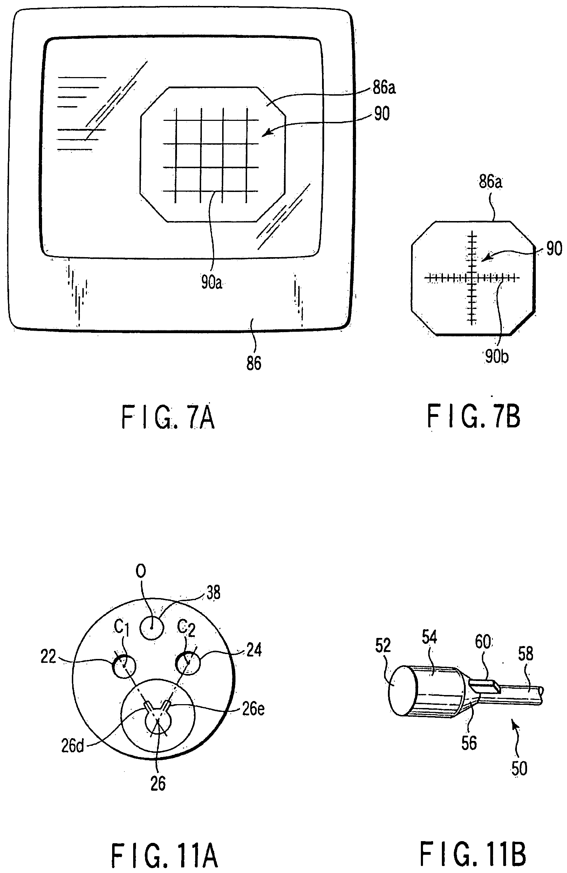

As shown in FIG. 7, the optical observation monitor 86 includes a display screen 86a. On the display screen 86a, a dimension indicator 90 for an image optically observed by the endoscope 10 can be displayed. The dimension indicator 90 and an image picked up by a Charge Coupled Device (CCD) 102 (described below) can be displayed one superimposed upon the other. An example of the dimension indicator 90 includes a grid 90a as shown in FIG. 7A or a scale 90b as shown in FIG. 7B, which can be selectively or simultaneously displayed on the display screen 86a.

Referring back to FIG. 6, the ultrasonic endoscope 10 includes the ultrasonic transducer 52 (see FIGS. 2 and 3) for ultrasonic observation, and the CCD 102 for optical observation. The video processor 82 includes a CPU 104, a CCD drive signal control circuit 106, a CCD drive signal generating circuit 108, a video processing circuit 110, and a graphics memory 112. Coupled to the CPU 104 are the CCD drive signal control circuit 106 and the graphics memory 112. The graphics memory 112 stores the images (see FIGS. 7A and 7B) of the dimension indicator 90 to be displayed on the optical observation monitor 86. Coupled to the CCD drive signal control circuit 106 is the CCD drive signal generating circuit 108 coupled to the CCD 102. Coupled to the CCD drive signal generating circuit 108 is the video processing circuit 110. Coupled to the graphics memory 112 and video processing circuit 110 is the optical observation monitor 86.

The ultrasonic observation device 84 includes: a transmission/reception control circuit 114 electrically coupled to the CPU 104 of the video processor 82; a transmission/reception circuit 116; a detector circuit 118; an analog to digital (A/D) conversion circuit 120; and a digital scan converter (DSC) 122. Coupled to the transmission/reception control circuit 114 is the transmission/reception circuit 116 coupled to the ultrasonic transducer 52. Coupled to the transmission/reception circuit 116 is the detector circuit 118. Coupled to the detector circuit 118 is the A/D conversion circuit 120. Coupled to the A/D conversion circuit 120 is the DSC 122. Coupled to the DSC 122 is the ultrasonic observation monitor 88.

Next, a description will be given of the case where using the ultrasonic endoscope 10 having a configuration as described above, an optically observed image and an ultrasonically observed image are displayed on the optical observation monitor 86 and the ultrasonic observation monitor 88, respectively.

Via the CCD drive signal control circuit 106, the CPU 104 drives the CCD drive signal generating circuit 108 and controls the CCD 102. The signal corresponding to an image picked up by the CCD 102 is input to the video processing circuit 110 via the CCD drive signal generating circuit 108. The video processing circuit 110 outputs this input image signal to the optical observation monitor 86.

In order to oscillate the ultrasonic transducer 52, a signal is input to the CPU 104 by a switch (not shown). The CPU 104 oscillates the ultrasonic transducer 52 via the transmission/reception control circuit 114 and transmission/reception circuit 116. On the other hand, a signal received by the ultrasonic transducer 52 is input to the transmission/reception control circuit 114 and detector circuit 118 via the transmission/reception circuit 116. The signal input to the detector circuit 118 is digitized converted by the A/D conversion circuit 120 and the resultant signal is input to the DSC 122. The DSC 122 outputs an ultrasonically observed image to the ultrasonic observation monitor 88.

The signal input to the transmission/reception control circuit 114 via the transmission/reception circuit 116 of the ultrasonic observation device 84 is received by the CPU 104. The CPU 104 causes the optical observation monitor 86 to display the dimension indicator 90, which is the image stored in the graphics memory 112. Specifically, as indicated by sign F, the dimension indicator 90 is displayed on the optical observation monitor 86 via the transmission/reception control circuit 114, CPU 104, and graphics memory 112. In response to an ON-signal indicating the ultrasonic scanning, the dimension indicator 90, which is the image stored in the graphics memory 112, is displayed so as to be superimposed upon the image picked up by the CCD 102. This makes it possible to estimate the size, etc., of a tissue based upon the dimension indicator 90.

Subsequently, a signal directing the ultrasonic transducer 52 to cease oscillation is input to the CPU 104 by a switch (not shown). The CPU 104 stops the oscillation of the ultrasonic transducer 52 via the transmission/reception control circuit 114 and transmission/reception circuit 116. Consequently, the signal from the transmission/reception control circuit 114 to the CPU 104 is intercepted. Since the CPU 104 does not transmit a signal to the graphics memory 112, the image of the dimension indicator 90 in the graphics memory 112 disappears from the optical observation monitor 86.

Next, the operation of the ultrasonic endoscope 10 according to the first embodiment will now be explained.

The distal end of the insertion section 12 of the ultrasonic endoscope 10 is inserted into a target body cavity, in such as an internal organ. While a body wall BW of the area of interest (not shown) in the body cavity is displayed and optically observed on the optical observation monitor 86, the ultrasonic transducer 52 projecting from the distal end face of the distal end rigid portion 32 of the ultrasonic probe 50 is brought into contact with the body wall BW of the area of interest. At this time, since the ultrasonic transducer 52 projects from the distal end face of the insertion section 12, as shown in FIG. 2, a suitable distance is maintained from the objective lens 38 to the body wall BW being in contact with the ultrasonic transducer 52. Accordingly, the surface of the body wall BW that includes the area of interest can be optically observed while the ultrasonic transducer 52 is kept in contact with the body wall BW. In this condition, the ultrasonic transducer 52 of the ultrasonic probe 50 is caused to ultrasonically oscillate (i.e., ultrasonic scanning is started). At this time, the focal length FL for optically observing the body wall BW has been adjusted. This makes it possible to ultrasonically observe the inside of the body wall BW of the passage in the body cavity while optically observing the surface of the body wall BW.

It is assumed that the second working channel 24 is disposed in the same plane as that in which the scanning surface S of the ultrasonic transducer 52 is disposed, as shown in FIG. 3B. In this case, a surgical instrument projecting from the working channel opening 24a of the second working channel 24 can be optically observed. For example, when a distal end of a surgical instrument such as a needle is inserted into the body wall BW, the position of the distal end of the surgical instrument may be ultrasonically observed. This makes it possible to ultrasonically observe the distal end of the surgical instrument reaching a target area in the body wall.

When the ultrasonic transducer 52 of the ultrasonic probe 50 is in contact with the body wall in order to ultrasonically observe the body wall, the distance between the body wall and the objective lens 38 is nearly constant. At this time, the size of the area under observation can be easily estimated by properly displaying the dimension indicator 90, such as the grid 90a or scale 90b, on the optical observation monitor 86.

When another surgical instrument is inserted through the first working channel 22, the scanning surface S of the ultrasonic probe 50 is preferably altered. In this case, as shown in FIG. 3A, the ultrasonic transducer 52 of the ultrasonic probe 50 is rotated so that the central axis C₁ of the first working channel 22 passes through the scanning surface S of the ultrasonic transducer 52. In other words, the first working channel 22 is disposed in the same plane as that in which the scanning surface S of the ultrasonic transducer 52 is disposed.

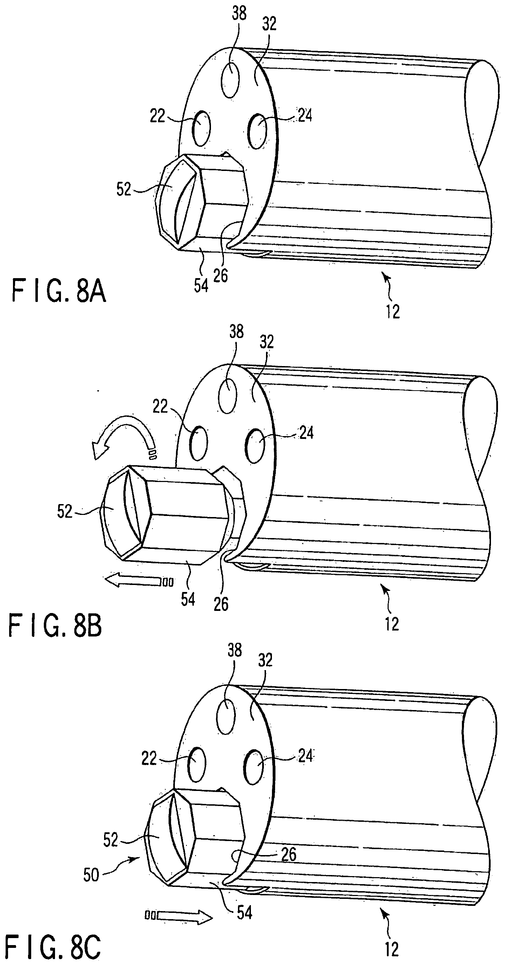

To rotate the ultrasonic probe 50, the holding portion 54 of the ultrasonic probe 50 requires temporary disengagement from the probe channel opening 26a. Therefore, the ultrasonic transducer 52 in the state shown in FIG. 8A is temporarily separated from the body wall BW. Then, the axially movable lever 62 is shifted from the state shown in FIG. 4C to the state shown in FIG. 4B so that the holding portion 54 projects from the probe channel opening 26a. In this condition, the rotation knob 72 is operated. Consequently, the transducer cable 58 receives the force, and the holding portion 54 at the distal end of the transducer cable 58 is rotated, as shown in FIG. 8B. Accordingly, the ultrasonic transducer 52 of the holding portion 54 can be rotated around the axis. In this condition, the movable lever 62 is returned to its original position, as shown in FIG. 8C. Consequently, the central axis C₁ of the first working channel 22 intersects the scanning surface S of the ultrasonic transducer 52, as shown in FIG. 3A.

Then, the ultrasonic transducer 52 of the ultrasonic probe 50 is brought into contact with a target area while optically viewed. At this time, projection of the surgical instrument from the working channel opening 22a of the first working channel 22 may be optically viewed. After the distal end of the surgical instrument is inserted into the body wall, the position of the surgical instrument is ultrasonically viewed.

The following benefits and advantages are obtained from the first embodiment described above.

The ultrasonic transducer 52 is fixed in such a position that it projects from the distal end face of the insertion section 12 of the ultrasonic endoscope 10. Accordingly, while the focal length FL required for optical observation from the distal end face of the insertion section 12 is maintained relative to the body wall BW including the target area, the same site can be ultrasonically observed. That is, substantially the same site can be observed both optically and ultrasonically.

When the ultrasonic transducer 52 is in contact with the body wall BW, the focal length FL between the body wall BW and the objective lens 38 is fixed. The fixed focal length FL for optical observation allows the determination of the size of the area in focus. Accordingly, the dimension indicator 90 for estimating the dimensions of the area being observed can be displayed on the optical observation monitor 86 so as to be superimposed on the image being optically observed, and the dimensions of the area of interest can be specified.

The ultrasonic transducer 52 can be rotated in relation to the distal end rigid portion 32 of the insertion section 12. Particularly, the ultrasonic transducer 52 is rotatable between the position where the central axis C₁ of the first working channel 22 is disposed on the central axis of the ultrasonic transducer 52 and the position where the central axis C₂ of the second working channel 24 is disposed on the central axis of the ultrasonic transducer 52. That is, the ultrasonic transducer 52 is fixable at more than one angle by rotation. In addition, since the ultrasonic scanning surface (i.e., ultrasonic oscillating surface) S is located on the central axis of the ultrasonic transducer 52, either of the distal ends, or suchlike, of a surgical instrument projecting in the body wall from the first and second working channel 22 and 24 can be selectively viewed on the ultrasonic observation monitor 88. This makes it easy to view the distal ends or the like of the surgical instrument during a delicate surgical operation. It enhances the secure application of surgery.

In the foregoing, a description was given in the case where the holding portion 54 of the ultrasonic probe 50 has the shape of an approximately regular hexagonal prism. However, any other approximately regular polygonal pyramid such as an approximately regular hexagonal pyramid is also suitable. In this case, the cross-section becomes smaller toward the proximal end of the ultrasonic probe 50. This allows a smaller size of the probe channel opening 26a of the distal end rigid portion 32 of the insertion section 12, in comparison with the case where the holding portion 54 has the shape of an approximately regular hexagonal prism.

Incidentally, the dimension indicator 90 can also be displayed on the optical observation monitor 86 in such a manner as described below.

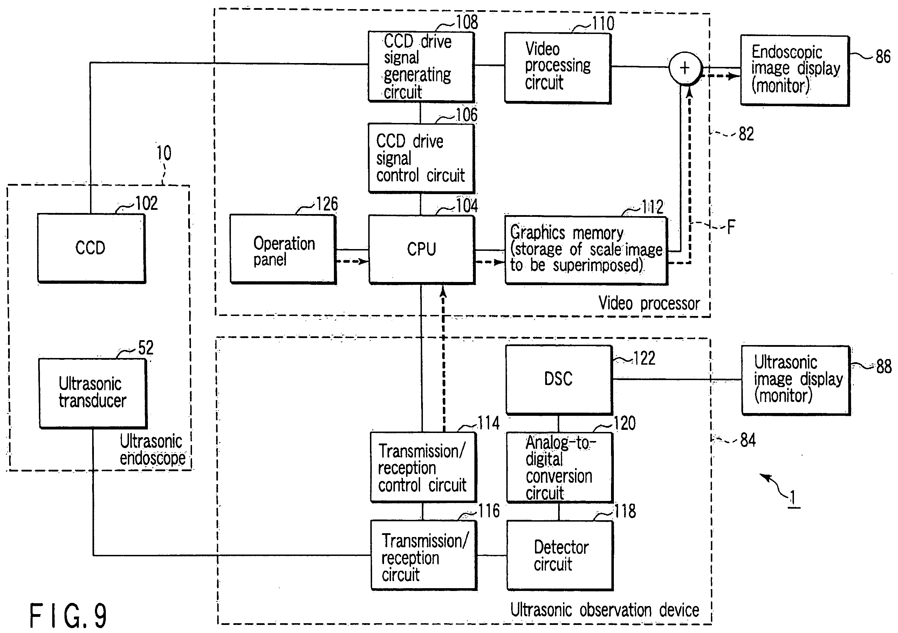

In addition to the configuration shown in FIG. 6, also an operation panel 126 is coupled to the CPU 104 of the video processor 82, as shown in FIG. 9. The operation panel 126 is capable of switching between the displayed and non-displayed states of the dimension indicator 90. In this case, the dimension indicator 90 can be displayed regardless of whether ultrasonic observation using the ultrasonic transducer 52 takes place or not.

Further, the dimension indicator 90 may be displayed in the manner described below.

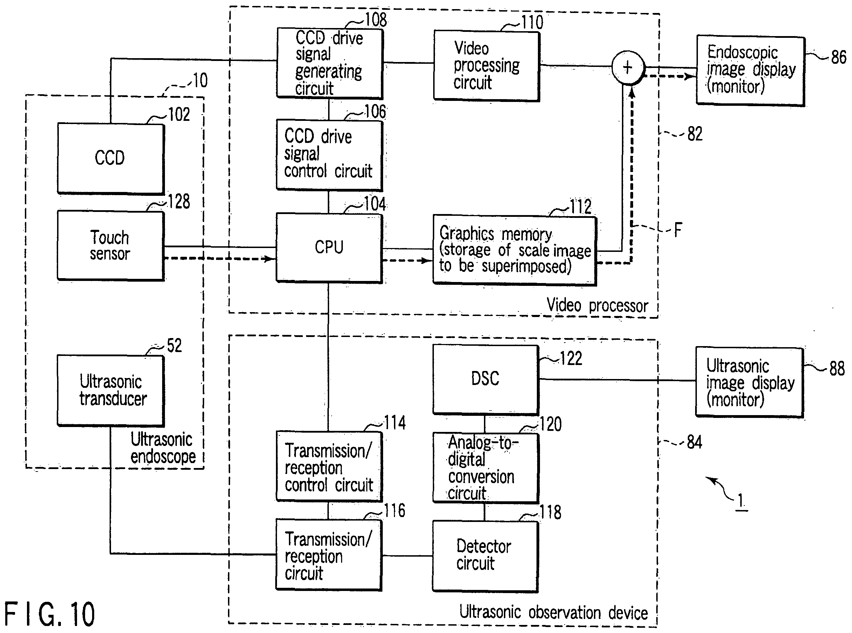

As shown in FIG. 10, the endoscope 10 incorporates a touch sensor 128, which is coupled to the video processor 82. Upon sensing a touch, the touch sensor 128 supplies a signal to the CPU 104 so as to allow switching between the displayed and non-displayed states of the dimension indicator 90. In this case, the dimension indicator 90 can be displayed regardless of whether ultrasonic observation using the ultrasonic transducer 52 takes place or not.

It is also preferable that the operation panel 126 shown in FIG. 9 be coupled to the CPU 104 of the video processor 82. Accordingly, in response to a touch sensed by the touch sensor 128, switching between the displayed and non-displayed states of the dimension indicator 90 may be achieved through the operation panel 126.

### (Second Embodiment)

A second embodiment, which is a modified example of the first embodiment, will now be described with reference to FIGS. 11A and 11B, in which like numbers indicate like elements and the explanation thereof will be omitted.

As shown in FIG. 11A, the probe channel opening 26a of the ultrasonic probe channel 26 of the endoscope 10 has a pair of angle fixing grooves (i.e., recesses) 26d and 26e. The angle fixing grooves 26d and 26e extend in the direction of the center C₁ of the first working channel 22 and in the direction of the center C₂ of the second working channel 24, respectively.

As shown in FIG. 11B, formed on the semispherical part 56 of the ultrasonic probe 50 is a flat rib (i.e., projection) 60 radially extending from the central axis of the ultrasonic probe 50. When the ultrasonic probe 50 rotates, the rib 60 fits into the angle fixing groove 26d or 26e, thereby positioning the holding portion 54 of the ultrasonic probe 50 in relation to the ultrasonic probe channel 26.

In this case, instead of a polygonal prism, such as an approximately regular hexagonal prism, or an approximately polygonal pyramid, the holding portion 54 may be an approximate cylinder, an approximately truncated cone, or the like. This also applies to the probe channel opening 26a of the ultrasonic probe channel 26.

Further, as shown in FIG. 11B, the rib 60 is formed on the semispherical part 56. Setting the rib 60 in a required position between the holding portion 54 and the semispherical part 56, the projected position of the ultrasonic transducer 52 from the distal end face of the insertion section 12 can be properly defined. In addition, the axial length of the rib 60 is appropriately set.

In the second embodiment, a description was given exemplifying the case where only one rib 60 is formed on the ultrasonic probe 50. However, it may be preferable that a plurality of ribs 60 be formed, for example, in the circumferential direction of the semispherical part 56. In this case, it is necessary that more than one pair of angle fixing grooves (i.e., more than two angle fixing grooves) be formed.

Further, the relation between the angle fixing grooves 26d and 26e and the rib 60 may be reversed. That is, projections may replace the angle fixing grooves, and recesses may replace the projections in order to serve as ribs. Such a design also enables the ultrasonic transducer 52 to be fixed at two or more angles by rotation.

The rib 60 is not limited to a flat shape but may equally be a member having a curved shape. In this case, the angle fixing grooves 26d and 26e should also be of a corresponding shape so as to be engaged with the rib 60. As long as the distal end of the ultrasonic probe 50 may be engaged or disengaged with the distal end of the insertion section 12 in a desired condition by specifying the shape, any shape can be used.

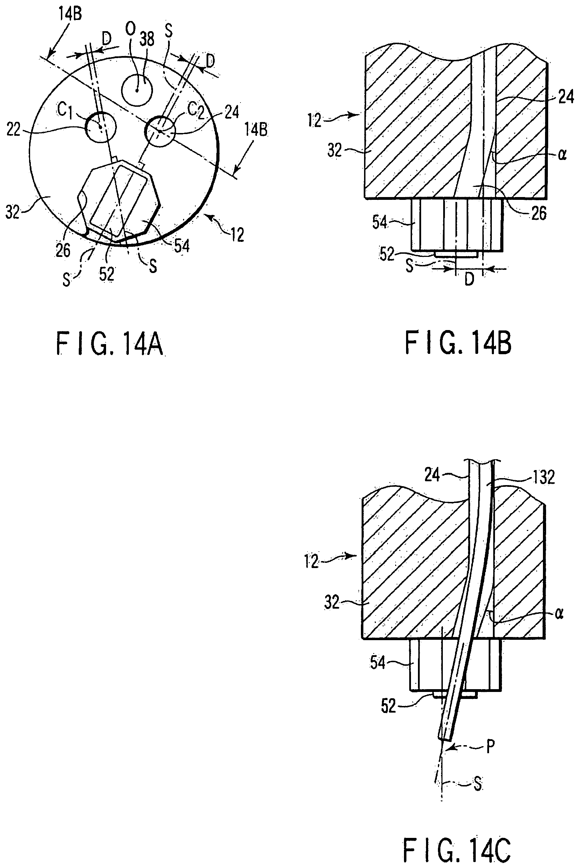

As in a fifth embodiment described below (see FIG. 14A), if the central axis (i.e., ultrasonic scanning surface) S of the ultrasonic transducer 52 needs to be displaced by distance D, for example, from the center C₁ of the first channel 22, the fixed groove 26d may be displaced or another fixed groove may be formed adjacent to the fixed groove 26d shown in FIG. 11A.

### (Third Embodiment)

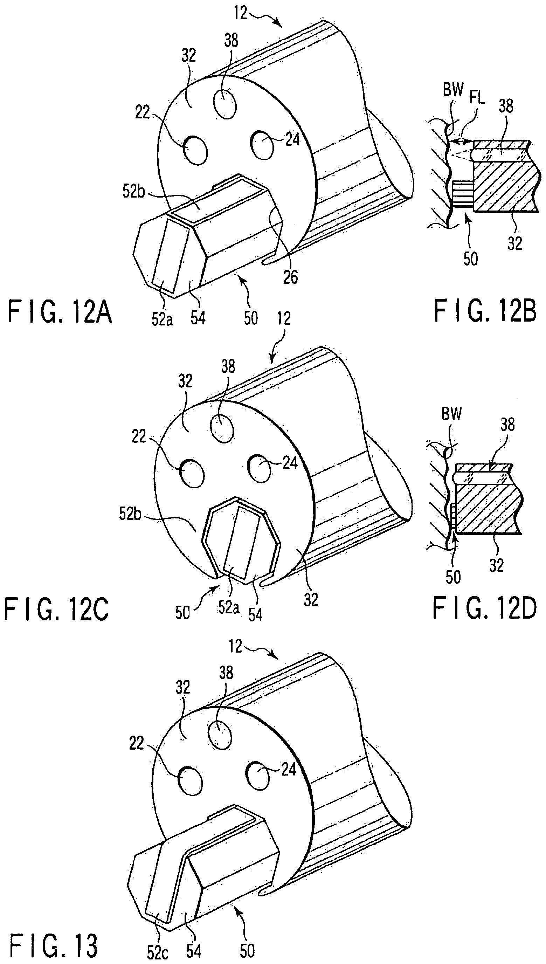

A third embodiment, which is a modified example of the first and second embodiments, will now be described with reference to FIGS. 12A to 12D, in which like numbers indicate like elements and the explanation thereof will be omitted.

As shown in FIG. 12A, disposed in the holding portion 54 of the ultrasonic probe 50 are a first ultrasonic transducer 52a and a second ultrasonic transducer 52b. The first ultrasonic transducer 52a is disposed in the same manner as the first embodiment. The second ultrasonic transducer 52b is disposed on one side face of the holding portion 54. Specifically, the second ultrasonic transducer 52b is disposed near the first working channel 22 and second working channel 24.

As in the first embodiment, the ultrasonic probe 50 according to the third embodiment is movable in the direction of and rotatable around its axis. As described in the first embodiment, the holding portion 54 of the ultrasonic probe 50 suitably projects from the distal end rigid portion 32 of the insertion section 12 of the endoscope 10. The length of the projection of the holding portion 54 from the distal end rigid portion 32 of the ultrasonic probe 50 is adjusted to the focal length FL of the objective lens 38, as shown in FIG. 12B. At this time, the second ultrasonic transducer 52b projects from the distal end rigid portion 32 and is directed toward the first working channel 22 or second working channel 24.

In the third embodiment, as shown in FIGS. 12C and 12D, the first ultrasonic transducer 52a of the ultrasonic probe 50 is pulled into a plane almost the same as that of the face of the distal end rigid portion 32 of the insertion section 12 of the endoscope 10. Therefore, in the third embodiment, the stroke of the axial movement of the ultrasonic probe 50 is greater than that in the first embodiment, or the axial length of the holding portion 54 of the ultrasonic probe 50 is shorter than that in the first embodiment. Such a configuration allows the second ultrasonic transducer 52b to be switched between the exposed position and the accommodated position inside the distal end rigid portion 32 of the insertion section 12.

The following benefits and advantages are obtained from the third embodiment.

The second ultrasonic transducer 52b disposed on the side face of the holding portion 54 of the ultrasonic probe 50 can be selectively fixed in the projected position or the accommodated position inside the distal end rigid portion 32 of the insertion section 12 of the endoscope 10. Accommodating the second ultrasonic transducer 52b inside the distal end rigid portion 32 allows a shorter length for the hard portion that is the sum of the length of the distal end rigid portion 32 and the length of the holding portion 54 of the ultrasonic probe 50 projecting from the distal end rigid portion 32, than that in the first embodiment. The short hard portion is less apt to bend when being introduced in a passage inside a body cavity. Accordingly, insertion efficiency is improved.

The first ultrasonic transducer 52a or second ultrasonic transducer 52b can be selectively oscillated or they can be simultaneously oscillated. This allows selective or simultaneous display of ultrasonically observed images on the monitor 88. Displaying both the ultrasonically observed images allows observation in a wider range, and hence ultrasonic observation at a larger scanning angle, than the case of displaying only one of the images.

By projecting the first ultrasonic transducer 52a of the ultrasonic probe 50 from the distal end of the insertion section 12, the vicinity of an area being ultrasonically observed can also be optically observed at the same time, as described in the first embodiment.

Incidentally, even if the second ultrasonic transducer 52b is oscillated when the holding portion 54 of the ultrasonic probe 50 is accommodated inside the distal end rigid portion 32 of the insertion section 12, an ultrasonically observed image cannot be obtained from the second ultrasonic transducer 52b. In addition, this condition does not allow the first ultrasonic transducer 52a to obtain an ultrasonically observed image and an optically observed image, because the body wall is too close to the first ultrasonic transducer 52a in relation to the focal length required for the optical observation such that it is impossible to simultaneously observe substantially the same area. This also applies to the configurations of conventional ultrasonic endoscopes.

### (Fourth Embodiment)

A fourth embodiment, which is a modified example of the third embodiment, will now be described with reference to FIG. 13, in which like numbers indicate like elements and the explanation thereof will be omitted.

In the fourth embodiment, the first ultrasonic transducer 52a and second ultrasonic transducer 52b described in the third embodiment are connected so as to be integrated, as shown in FIG. 13. That is, a single ultrasonic transducer 52c extends from the distal end of the holding portion 54 to one sidewall thereof. Other features of the configuration and operations are identical to those in the third embodiment.

This ultrasonic transducer 52c is controllable in three modes: oscillating only a distal end portion (i.e., scanning), oscillating only a side-face portion, and oscillating both of them. This allows selective ultrasonic observation using the distal end portion, side-face portion, or both, of the ultrasonic transducer 52c.

### (Fifth Embodiment)

Next, a fifth embodiment, which is a modified example of the first to fourth embodiments, will now be described with reference to FIGS. 14A to 15B, in which like numbers indicate like elements and the explanation thereof will be omitted.

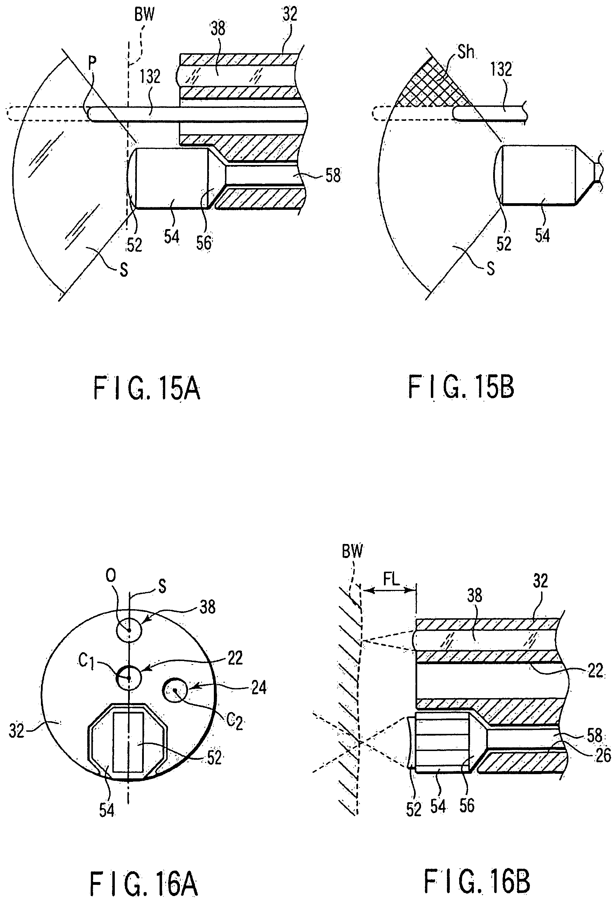

On the scanning surface of the ultrasonic transducer 52 described in each of the first to fourth embodiments are the center C₁ of the first working channel and the center C₂ of the second working channel 24. When a surgical instrument 132 is inserted in an ultrasonic observation area, as shown in FIG. 15B, the surgical instrument 132 reflects ultrasonic waves. Consequently, ultrasonic waves from the ultrasonic transducer 52 do not reach a site Sh that is beyond the surgical instrument 132. This makes it difficult to obtain an ultrasonically observed image of a site beyond the surgical instrument 132. However, the configuration described below makes it easy for an ultrasonic wave to reach a site located far away from the ultrasonic transducer 52, as shown in FIG. 15A. As a result, the ultrasonically observed image of the site can be obtained.

As shown in FIG. 14A, there is a displacement D between the central axis (ultrasonic scanning surface) S of the ultrasonic transducer 52 and the center C₁ of the first channel 22. There is also a displacement D between the central axis (ultrasonic scanning surface) S of the ultrasonic transducer 52 and the center C₂ of the second channel 24.

Further, as shown in FIG. 14B, the distal end of the second working channel 24 slightly inclines, for example, by an angle α with respect to the axis of the insertion section 12. The central axis C₂ of the second working channel 24 intersects the central axis (ultrasonic scanning surface) S of the ultrasonic transducer 52 outside the insertion section 12 of the endoscope 10. FIG. 14B illustrates the case of the second working channel 24, while the first working channel 22 has the same configuration as the second working channel 24.

Next, a description will be given of the operation of the ultrasonic endoscope 10 according to the fifth embodiment.

As shown in FIG. 14C, the surgical instrument 132 is obliquely introduced from, for example, the distal end of the second channel 24 toward an area S to be ultrasonically observed. Consequently, the surgical instrument 132 intersects the central axis (scanning surface) S of the ultrasonic transducer 52 close to a predetermined position P. The inclination angle α between the scanning surface S of the ultrasonic transducer 52 and the distal end of the second channel 24 is small. This does not mean that only the area where the surgical instrument 132 intersects the scanning surface of the ultrasonic transducer 52 is ultrasonically observable. On the contrary, an image is displayed on the ultrasonic observation monitor 88 such that the density at the intersecting position P of the surgical instrument and ultrasonic scanning surface S is highest and the density gradually decreases further from the intersecting position P. This makes it possible to view the surgical instrument 132 not only at the intersecting position P of the surgical instrument and ultrasonic scanning surface S but also over the entire observation area S, depending on the use condition.

The surgical instrument 132 substantially totally reflects only ultrasonic waves at the intersecting position P of the surgical instrument 132 and scanning surface S, and does not reflect ultrasonic waves in other sites. This minimizes interception of ultrasonic oscillation transmitted from the ultrasonic transducer 52, and enables ultrasonic waves to reach sites located farther than the intersecting position P of the surgical instrument 132 and scanning surface S. Accordingly, sites located far away from the ultrasonic transducer 52 can be displayed on the monitor 88.

In the fifth embodiment, a description was given exemplifying the case where the surgical instrument 132 is inserted in the second working channel 24. However, the same may be applied to a surgical instrument inserted in the first working channel 22.

The following benefits and advantages are obtained from the fifth embodiment.

The working channels 22 and 24 are slightly bent near the channel openings 22a and 24a respectively. Therefore, when inserted in each working channel 22, 24, the surgical instrument 132 projects from the channel opening 22a, 24a while in contact with the internal face of the channel opening 22a, 24a. This prevents backlash (i.e., play) of the surgical instrument 132 against the working channels 22 and 24. This also causes the central axis of the surgical instrument 132 to incline when the surgical instrument 132 projects from each channel opening 22a, 24a, thus enabling the surgical instrument 132 to intersect the scanning surface S of the ultrasonic transducer 52. Consequently, an ultrasonic wave is transmitted farther than the intersecting position P with the surgical instrument 132. Accordingly, a satisfactory ultrasonically observed image can be obtained even if the target area is beyond the surgical instrument 132.

### (Sixth Embodiment)

A sixth embodiment, which is a modified example of the first to fourth embodiments, will now be described with reference to FIGS. 16A and 16B, in which like numbers indicate like elements and the explanation thereof will be omitted.

Unlike the convex type ultrasonic transducer 52 of the distal end of the ultrasonic probe 50 according to each of the first to third embodiments, the ultrasonic transducer 52 according to the sixth embodiment is of a concave type, as shown in FIG. 16B. The first working channel 22 and the objective lens 38 are disposed on the central axis S of the ultrasonic transducer 52 as shown in FIG. 16A. The first working channel 22 is defined almost in the middle of the distal end rigid portion 32.

Accordingly, focusing positions for ultrasonic observation and optical observation coincide by adjusting the concave shape of the ultrasonic transducer 52 or the focal length. This allows ultrasonic observation and optical observation of substantially the same area. Additionally, the first working channel 22 is defined in the middle, which makes it possible to optically and ultrasonically view a surgical instrument projecting from the distal end of the first working channel 22.

Incidentally, since ultrasonic oscillation resists passage of gases such as air, any space between the ultrasonic transducer 52 and a body wall needs to be filled with a member, such as an abdominal cavity fluid or physiological saline, that satisfactorily transmits ultrasonic oscillation.

The following benefits and advantages are obtained from the sixth embodiment.

The concave type ultrasonic transducer 52 makes the focal length and focal position for ultrasonic observation and those for optical observation substantially the same, thus allowing both ultrasonic observation and optical observation of the same area.

Additionally, disposing the first working channel 22 between the objective lens 38 and ultrasonic probe channel 26 makes it possible to optically and ultrasonically view the position, etc. of a surgical instrument relative to a body wall BW.

### (Seventh Embodiment)

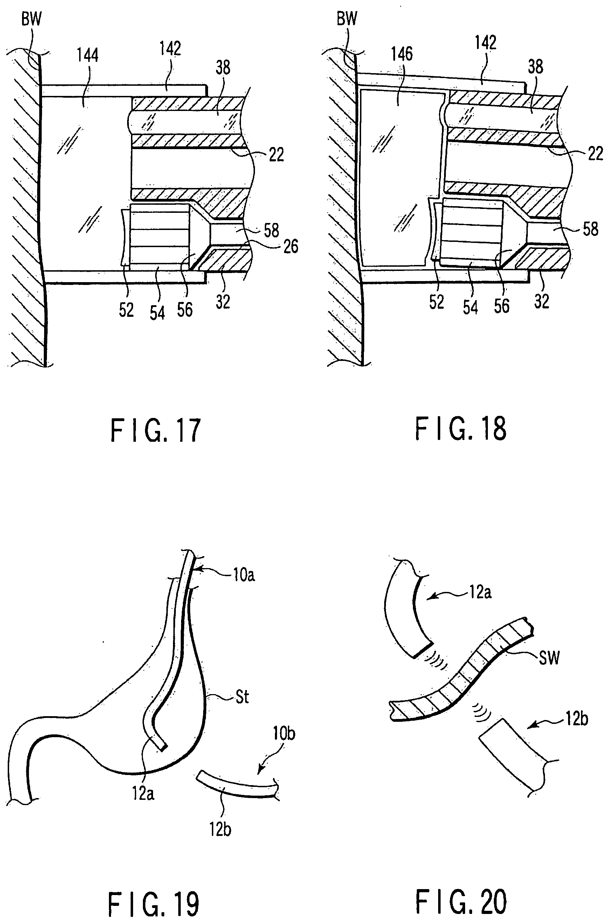

A seventh embodiment, which is a modified example of the sixth embodiment, will now be described with reference to FIG. 17, in which like numbers indicate like elements and the explanation thereof will be omitted.

As shown in FIG. 17, a cap 142 is fixed to the periphery of the distal end rigid portion 32 of the insertion section 12 of the ultrasonic endoscope 10. It is preferable that the cap 142 be transparent. The cap 142 may be made of a flexible material such as silicone so that the distal end of the cap 142 is properly deformable when in contact with the body wall BW and may be brought into close contact with the body wall BW.

Next, a description will be given of the operation of the ultrasonic endoscope 10 according to the seventh embodiment.

As shown in FIG. 17, the distal end of the insertion section 12 of the ultrasonic endoscope 10 is introduced into a passage inside a body cavity, with the cap 142 fixed to the distal end of the insertion section 12. Then, the distal end of the cap 142 is pressed against a body wall. Consequently, the body wall BW, the internal face of the cap 142, and the face of the distal end rigid portion 32 define an empty space. In this condition, a transparent liquid 144 such as a physiological saline is injected into this space through the working channel 22, so that the space is filled with the liquid (physiological saline) 144. For example, the physiological saline should be transparent and satisfactorily capable of transmitting ultrasonic oscillation. Accordingly, the endoscope 10 allows ultrasonic observation of the vicinity of the surface of the body wall BW in the direction of depth as well as optical observation of the surface of the body wall BW.

The following benefits and advantages are obtained from the seventh embodiment.

The cap 142 is made of a flexible material, which is deformable when brought into contact with the body wall BW. Therefore, even though the cap 142 projects from the distal end rigid portion 32, the cap 142 does not block the introduction of the insertion section 12 into a passage inside a body cavity.

The medium 144, such as physiological saline, which is transparent and capable of transmitting ultrasonic oscillation is injected using the channel 22. This makes it possible to obtain an optically observed image using the transparent medium 144 as well as an ultrasonically observed image. At this time, as described in the sixth embodiment, the focused position of an ultrasonically observed image and that of an optically observed image substantially coincide. Accordingly, an ultrasonically observed image and optically observed image of substantially the same area can be obtained.

### (Eighth Embodiment)

An eighth embodiment, which is a modified example of the seventh embodiment, will now be described with reference to FIG. 18, in which like numbers indicate like elements and the explanation thereof will be omitted.

The space defined by a body wall BW, the internal circumferential wall of the cap 142, and the face of the distal end rigid portion 32, is not necessarily filled with the liquid medium 144 such as a physiological saline injected through the working channel 22, as described in the seventh embodiment.

Instead, a transparent block 146, for example, may be suitably disposed in this space so as to be in close contact with the ultrasonic transducer 52, as shown in FIG. 18. In terms of ultrasonic transmission, light transmission, biocompatibility, contact with a body cavity, mechanical strength, etc, it is preferable that the block 146 be made of, for example, a material from which soft contact lenses are made.

Preferable examples of the material for the block 146 include a hydrous gel polymer of polyhydroxy ethyl methacrylate (PHEMA), an anhydrous silicone hydogel (SH), a hydrous gel polymer of agar, and an anhydrous epoxy resin.

Since the PHEMA contains water, it excels in ultrasonic transmission, but is not very strong. This enables certain types of surgical instrument to pierce through the block 146 when projecting from the working channel opening 22a disposed in contact with the block 146. Consequently, the surgical instrument projecting from the working channel 22 and piercing through the block 146 also appears on the ultrasonic image.

Since SH is nonionic, it excels in stain resistance. For instance, even if the surface of the block 146 comes into contact with a waste material remaining on a wall of the alimentary canal, a satisfactory optical visual field can be obtained through the block 146.

Agar is a biocompatible material. Even in the event that agar is deposited within a body cavity, it does not affect any tissue. In addition, agar is not very strong. This enables certain types of surgical instrument to pierce through the block 146 when projecting from the working channel opening 22a disposed in contact with the block 146. Consequently, the surgical instrument projecting from the working channel 22 and piercing through the block 146 also appears on the ultrasonic image.

Some epoxy resins excel in chemical resistance. The block 146 using such a chemical-resistant epoxy resin is reusable after disinfection or sterilization.

### (Ninth Embodiment)

Next, a ninth embodiment will be described with reference to FIGS. 19 to 21D.

As shown in FIG. 19, the ninth embodiment uses two ultrasonic endoscopes (first and second endoscopes) 10a and 10b. The first endoscope 10a is inserted in a passage inside a body cavity, such as the stomach St, through the mouth. On the other hand, the second endoscope 10b is inserted in the body cavity outside the stomach St by way of the skin. Then, the first endoscope 10a and second endoscope 10b are disposed opposite to each other with the stomach wall SW therebetween, as shown in FIG. 20. In this case, for example, an ultrasonic transducer for ultrasonic wave transmission (not shown) is disposed at the distal end of an insertion section 12a of the first endoscope 10a, whereas an ultrasonic transducer for ultrasonic wave reception (not shown) is disposed at the distal end of an insertion section 12b of the second endoscope 10b. Then, the insertion section 12a of the first endoscope 10a and the insertion section 12b of the second endoscope 10b may be moved relative to one another so as to adjust to the position where reception of ultrasonic oscillation is strongest.

This facilitates alignment of the distal ends of the insertion sections 12a and 12b of the endoscopes 10a and 10b so that the distal ends face each other. In addition, when a needle pierces through a stomach wall SW (i.e., body cavity wall), the distal end of the needle having pierced through the wall is brought into contact with the distal end of the insertion section 12b of the second endoscope 10b. This improves the safety of a surgical operation. Further, this facilitates transfer of a thread while suturing.

In the ninth embodiment, the first endoscope 10a incorporates the ultrasonic transducer for transmission while the second endoscope 10b incorporates the ultrasonic transducer for reception. However, it is also preferable that each endoscope 10a, 10b incorporate both an ultrasonic transducer for transmission and one for reception.

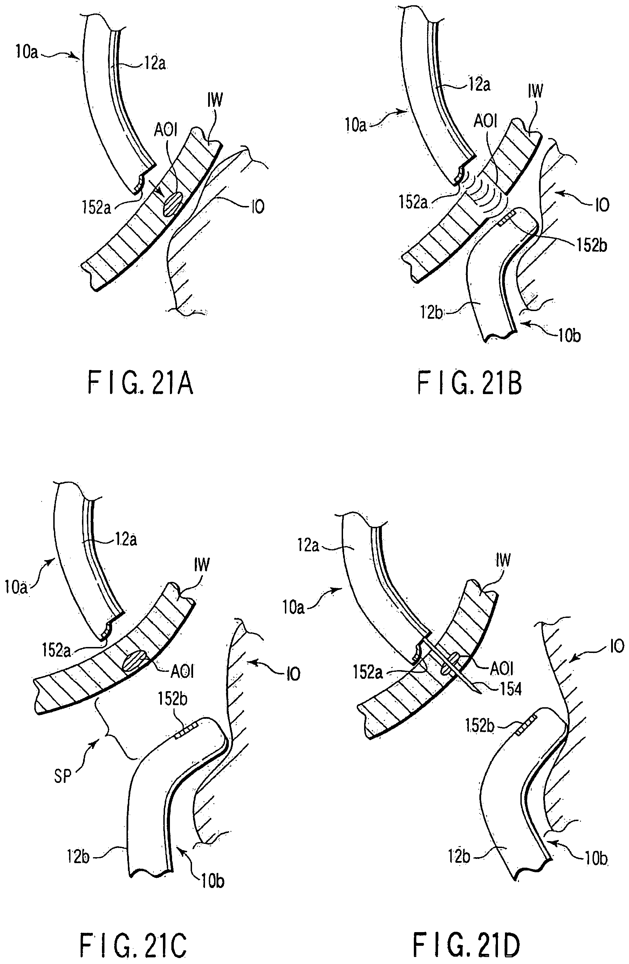

Referring to FIGS. 21A to 21D, other related operations using the two endoscopes 10a and 10b will now be described.

As shown in FIG. 21A, beyond an intestinal wall IW may be an internal organ 10 that must not be injured. In such a case, the ultrasonic endoscope 10a is introduced into the passage inside the body cavity through the mouth. Then, the area of interest AOI is viewed through ultrasonic observation.

Subsequently, the ultrasonic endoscope (or ultrasonic probe) 10b is introduced by way of the skin. The distal end of the insertion section 12a of the endoscope 10a introduced through the mouth and the distal end of the insertion section 12b of the endoscope 10b introduced by way of the skin are disposed opposite to each other with the intestinal wall IW therebetween. As shown in FIG. 21B, an ultrasonic transducer 152a at the distal end of the insertion section 12a of the ultrasonic endoscope 10a introduced through the mouth is used for transmission, and an ultrasonic transducer 152b at the distal end of the insertion section 12b of the ultrasonic endoscope 10b introduced by way of the skin for reception. At this time, attempts are made to determine the position where the signal received by the endoscope 10b introduced by way of the skin is strongest. The position of the strongest reception indicates the alignment where the two ultrasonic transducers 152a and 152b face each other. At this time, the distal end of the insertion section 12b of the endoscope 10b introduced by way of the skin pushes away the internal organ IO that must not be injured.

The ultrasonic endoscope 10b introduced by way of the skin is further separated from the area of interest AOI. As a result, a space SP is defined between the intestinal wall IW and the internal organ IO, as shown in FIG. 21C.

As shown in FIG. 21D, even in the case where a needle 154 pierces through the intestinal wall IW in this condition, the ultrasonic transducer 152b of the ultrasonic endoscope 10b disposed in the opposite position prevents the distal end of the needle 154 from coming into contact with other sites or injuring these sites.

The following benefits and advantages are obtained from the ninth embodiment.

The transmission and reception of ultrasonic oscillation makes it easier to align the ultrasonic transducer 152a of the endoscope 10a and the ultrasonic transducer 152b of the endoscope 10b (or ultrasonic probe) in opposition. Accordingly, a space SP can be defined between the body wall and the ultrasonic transducer 152b. In addition, the surgical instrument 154 piercing toward the ultrasonic transducer 152b prevents other sites from being injured.

### (Tenth Embodiment)

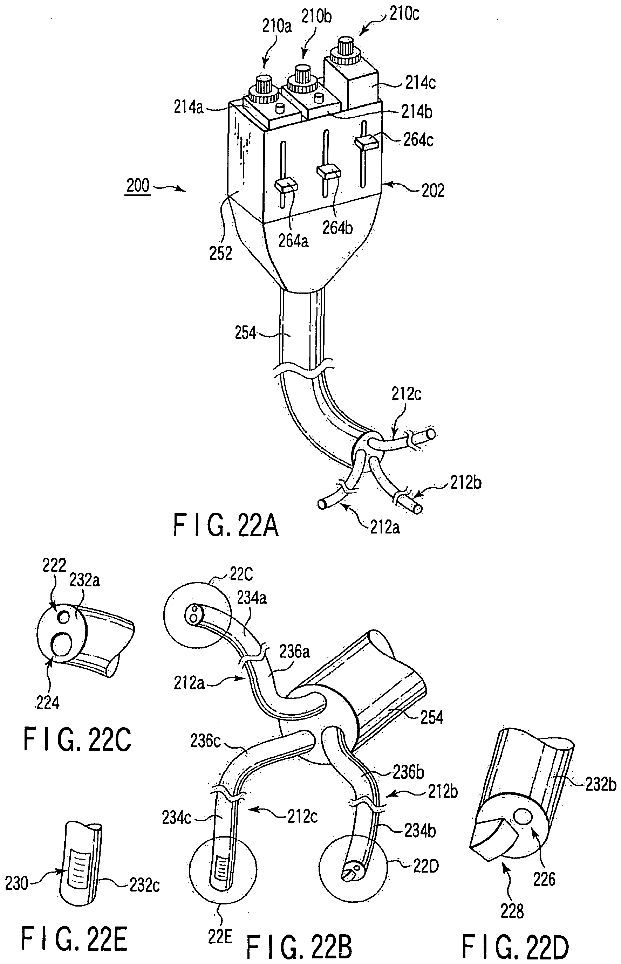

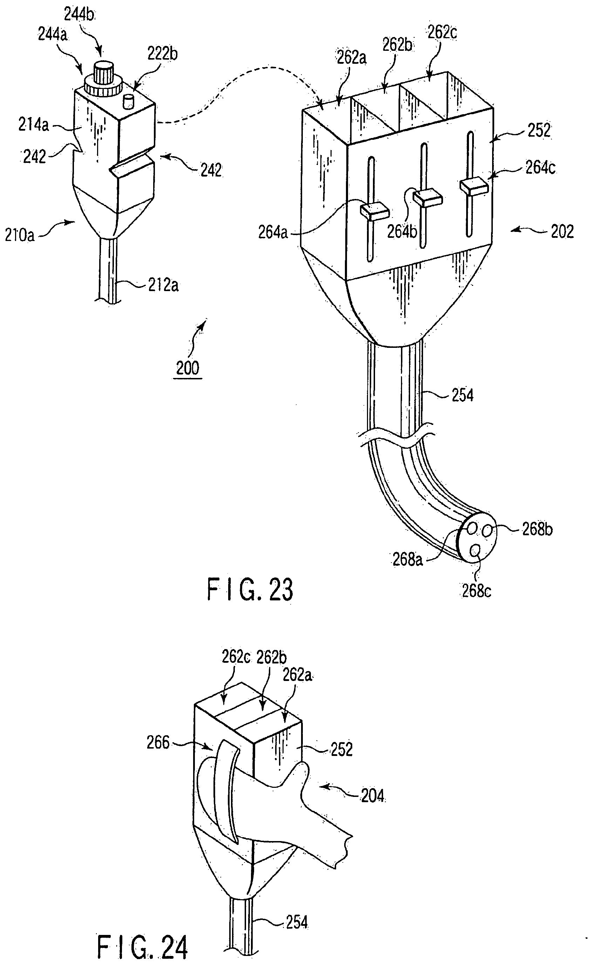

Next, a tenth embodiment will be described with reference to FIGS. 22A to 25C.

As shown in FIGS. 22A to 22E, an endoscopic system 200 includes a main body case 202, an optical observation endoscope 210a, an ultrasonic observation endoscope 210b, and an ultrasonic probe 210c.

The optical observation endoscope 210 shown in FIGS. 22A to 22C has a long thin insertion section 212a, and an operation section 214a disposed at the proximal end of the insertion section 212a. A working channel (forceps channel) 222 and an optical observation system incorporating an objective lens 224 are inserted in the insertion section 212a and the operation section 214a. The insertion section 212a includes a distal end rigid portion 232a, a bending portion 234a, and a flexible tubular portion 236a. An objective lens 238 and a channel opening 222a of the working channel 222 are disposed parallel to each other in the distal end rigid portion 232a.

The operation section 214a of the optical observation endoscope 210a is approximately rectangular parallel-epipedic so as to be disposed in a slot 262a (described below) formed in the main body case 202. Engagement grooves (i.e., notches) 242 with which a slide lever 264a (described below) engages are formed in the external faces of the operation section 214a so as to be perpendicular to the axial direction of the insertion section 212a. These engagement grooves 242 are opposite to each other. Coaxially disposed on the upper face of the operation section 214a are a vertical angle knob 244a in the form of a wheel (i.e., dial) for vertically curving the bending portion 234a and a lateral angle knob 244b in the form of a wheel (i.e., dial) for laterally curving the bending portion. A forceps mouthpiece 222b is disposed on the operation section 214a and on the proximal end side opening of the working channel 222.

The ultrasonic observation endoscope 210b shown in FIGS. 22A, 22B, and 22D includes a thin long insertion section 212b, and an operation section 214b disposed at the proximal end of the insertion section 212b. A working channel (forceps channel) 226 and an ultrasonic observation system incorporating an ultrasonic transducer 228 are inserted in the insertion section 212b and the operation section 214b. The insertion section 212b includes a distal end rigid portion 232b, a bending portion 234b, and a flexible tubular portion 236b. The ultrasonic transducer 228 is disposed on the distal end rigid portion 232b, and the channel opening 226a of the working channel 226 is also formed in the distal end rigid portion 232b.

The configuration of the operation section 214b of the ultrasonic observation endoscope 210b is similar to the operation section 214a of the optical observation endoscope 210a.

The ultrasonic probe 210c includes a thin long insertion section 212c, and an operation section 214c disposed on the proximal end of the insertion section 212c. An ultrasonic observation system incorporating an ultrasonic transducer 230 is inserted in the insertion section 212c and operation section 214c. The insertion section 212c includes a distal end rigid portion 232c, a bending portion 234c, and a flexible tubular portion 236c. The ultrasonic transducer 230 is disposed on the face of the distal end rigid portion 232c.

The configuration of the operation section 214c of the ultrasonic probe 210 is similar to the operation section 214a of the optical observation endoscope 210a and the operation section 214b of the ultrasonic observation endoscope 210b. Since a channel is not formed in the ultrasonic probe 210c, the ultrasonic probe 210c is not provided with a forceps mouthpiece.

The main body case 202 includes a holding portion 252 and a sheath 254. The holding portion 252 is sectioned into three slots 262a, 262b, and 262c, and slide levers (i.e., parts for adjusting the lengths of the insertion sections 212a, 212b, and 212c projecting from the distal end of the sheath 254) 264a, 264b, and 264c are provided for the corresponding slots 262a, 262b, and 262c. The slide levers 264a, 264b, and 264c are vertically operable, as viewed from FIG. 22A.

The operation section 214a of the optical observation endoscope 210a is disposed in the first slot 262a, the operation section 214b of the ultrasonic observation endoscope 210b in the second slot 262b, and the operation section 214c of the ultrasonic probe 210c in the third slot 262c. A hand strap 266 is fixed to the back of the holding portion 252 of the main body case 202 and opposite the slide levers 264a, 264b, and 264c. This makes it easy for the user to hold the main body case 202. The slide levers 264a, 264b, and 264c engage with the engagement grooves 242 of the operation sections 214a, 214b, and 214c, respectively. Accordingly, as shown in FIG. 22A, the optical observation endoscope 210a, ultrasonic observation endoscope 210b, and ultrasonic probe 210c are movable along their respective axes by operating the slide levers 264a, 264b, and 264c, respectively.

The sheath 254 includes a first lumen 268a, a second lumen 268b, and a third lumen 268c. The insertion section 212a of the optical observation endoscope 210a is introduced in the first lumen 268a, the insertion section 212b of the ultrasonic observation endoscope 210b in the second lumen 268b, and the insertion section 212c of the ultrasonic probe 210c in the third lumen 268c. The distal end rigid portion 232a, bending portion 234a, or part of the flexible tubular portion 236a of the insertion section 212a of the optical observation endoscope 210a can be projected from the distal end of the first lumen 268a. The distal end rigid portion 232b, bending portion 234b, or part of the flexible tubular portion 236b of the insertion section 212b of the ultrasonic observation endoscope 210b can be projected from the distal end of the second lumen 268b. The distal end rigid portion 232c, bending portion 234c, or part of the flexible tubular portion 236c of the insertion section 212c of the ultrasonic probe 210c can be projected from the distal end of the third lumen 268c.

The internal diameters of the first, second, and third lumens 268a, 268b, and 268c may or may not be identical.

Next, operation of the endoscopic system 200 according to the tenth embodiment will be described.

The optical observation endoscope 210a, ultrasonic observation endoscope 210b, and ultrasonic probe 210 are disposed in the main body case 202. Then, the insertion sections 212a, 212b, and 212c are introduced into a passage inside a body cavity.

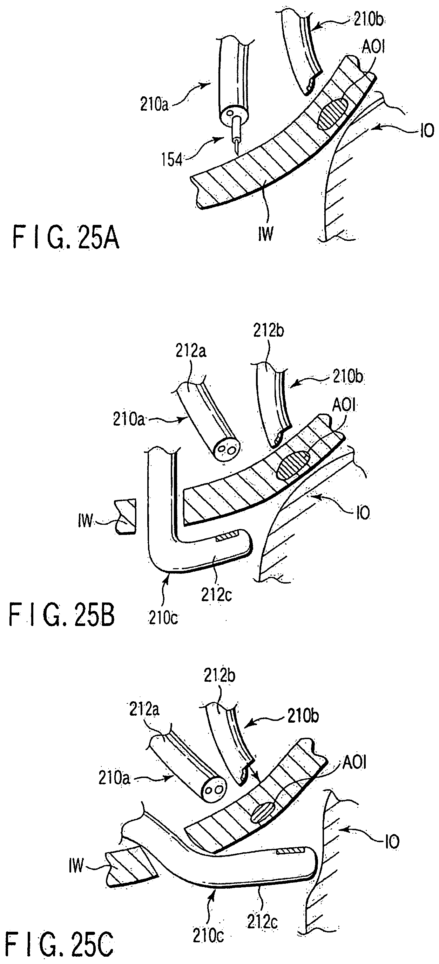

Subsequently, as shown in FIG. 25A, the area of interest AOI is ultrasonically observed with the ultrasonic observation endoscope 210b while the body wall is optically observed with the optical observation endoscope 210a. At this time, it is necessary to check whether sites or internal organs IO that must not be injured are located immediately near the area of interest AOI.

Next, a needle 154 pierces through an intestinal wall IW via the working channel 222 of the optical observation endoscope 210a. The site through which the needle has pierced is away from the area of interest AOI. Then, the distal end of the insertion section 212c of the ultrasonic probe 210c is introduced into this pierced site, as shown in FIG. 25B.

Then, as shown in the states of FIGS. 25B and 25C, the bending portion 234c of the ultrasonic probe 210c is curved and pushed so as to move away any site that is located near the intestinal wall IW and must not be injured. This defines a safe space that is not affected by the needle 154 piercing through the area of interest AOI.

Operating the slide levers 264a, 264b, and 264c allows movement of the insertion sections 212a, 212b, and 212c relative to one another. Thus, a required surgical operation can be performed by moving relative to each other the distal ends of the insertion sections 212a, 212b, and 212c of the optical observation endoscope 210a, ultrasonic observation endoscope 210b, and ultrasonic probe 210c of the main body case 202.

The following benefits and advantages are obtained from the tenth embodiment.

Accommodating the endoscopes and the like in the single main body case 202 allows the simultaneous use of such instruments. Disposing the plural operation sections 214a, 214b, and 214c near to one another makes their operation easier, and enables one user to simultaneously operate the endoscopes and the like alone.

Since the optical observation endoscope 210a, ultrasonic observation endoscope 210b, or ultrasonic probe 210c can be selected as required, a desired instrument can easily be brought into a required position. For example, this makes it possible to simultaneously carry out optical observation and ultrasonic observation from front and back.

Also, since the shapes of the operation sections 214a, 214b, and 214c of the optical observation endoscope 210a, ultrasonic observation endoscope 210b, and ultrasonic probe 210c are identical, each operation section may be disposed in any one of the slots 262a, 262b, and 262c. That is, the identical shape of the operation sections 214a, 214b, and 214c allows free setting of such instruments into the slots 262a, 262b, and 262c. The insertion sections 212a, 212b, and 212c of these instruments, needless to say, have outside diameters that allow the insertion of the insertion sections in corresponding lumens, 268a, 268b, and 268c.

The pair of engagement grooves 242 are formed in each operation section 214. Therefore, the endoscopes and the like may be disposed in the slots 262a, 262b, and 262c, regardless of the direction of the operation section of such instrument.

The hand strap 266 on the holding portion 252 of the main body case 202 makes it easy to integrally hold the plurality of instruments.

In the tenth embodiment, a description was given exemplifying the case where the holding portion 252 has three slots 262a, 262b, and 262c. However, the holding portion 252 may have two slots, in which case the sheath has two lumens.

### (Eleventh Embodiment)

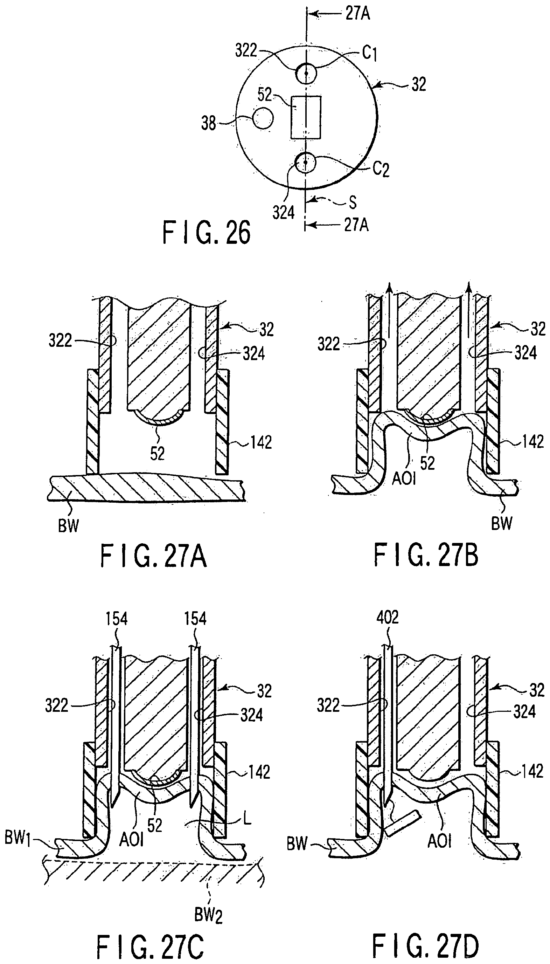

An eleventh embodiment, which is a modified example of the first embodiment, will now be described with reference to FIGS. 26 to 27D, in which like numbers indicate like elements and the explanation thereof will be omitted.

As shown in FIG. 26, the ultrasonic endoscope 10 includes, in the face of the distal end rigid portion 32 of the insertion section 12, the ultrasonic endoscope 52, a first suction passage (first suction channel) 322, a second suction passage (second suction channel) 324, and an objective lens 38. In particular, the ultrasonic transducer 52 is disposed near the center of the face of the distal end rigid portion 32 and between the first and second suction passages 322 and 324. The first and second suction passages 322 and 324 are on the scanning surface (i.e., oscillation surface) S located on the central axis of the ultrasonic transducer 52. The first suction passage 322 serves as a first working channel (forceps channel), and the second suction passage 324 as a second working channel (forceps channel). A detachable transparent cap 142 is fitted on the periphery of the distal end rigid portion 32.

Next, the operation of the ultrasonic endoscope 10 according to the eleventh embodiment will be described.

While a body wall BW is optically observed with the objective lens 38 (see FIG. 26) of the ultrasonic endoscope 10, the ultrasonic transducer 52 is pressed to the vicinity of a target area. Then, using the pair of suction passages 322 and 324, the living tissue is suctioned, as shown in FIG. 27A. Consequently, as shown in FIG. 27B, the body wall (i.e., the living tissue) BW is brought into close contact with the ultrasonic transducer 52. Accordingly, an ultrasonically observed image is stably shown. If the body wall has two layers, as indicated by signs BW₁ and BW₂, only the upper body wall BW₁, shown in FIG. 27C, is suctioned and lifted.

Then, while the suction continues, the needle 154 is passed through the suction passage 322 and pierces through the area of interest. At this time, as shown in FIG. 27C, the area of interest AOI of the body wall BW₁ is kept lifted by the suction, and consequently an abdominal cavity fluid L enters a space beyond the area of interest AOI. This makes it possible to estimate the positional relation between the body walls BW₁ and BW₂ through ultrasonic observation. By piercing the body wall BW₁ with the needle while checking the sharp point of the needle 154 as well as the positional relation between the body walls BW₁ and BW₂ through ultrasonic observation, the sharp point of the needle 154 can remain within the abdominal cavity fluid L. This easily prevents the sharp point of the needle 154 piercing the body wall BW₁ from reaching the body wall BW₂. Thus, the needle safely pierces the body wall BW₁ without injuring the body wall BW₂.

In order to prevent the sharp point of the needle 154 from projecting beyond the distal end of the cap 142, the movable range of the needle 154 may be adjusted in advance. Accordingly, as shown in FIG. 27C, the sharp point of the needle 154 piercing the body wall BW₁ is prevented from reaching the body wall BW₂. Thus, surgical operations to such specifications can easily be performed.

The following benefits and advantages are obtained from the eleventh embodiment.

The close contact of the ultrasonic transducer 52 and the body wall BW₁ by suction improves the display of an image shown on the ultrasonic observation monitor 88.

Since a target area to be pierced is suctioned to prevent it from moving, a surgical operation such as piercing the target area with the needle 154 is easier and more securely performed.

Incidentally, this allows surgical operations such as placing a T-bar 404 (described below, in the thirteenth embodiment) in the body wall BW₁ by means of a T-bar suturing device 402, as shown in FIG. 27D.

### (Twelfth Embodiment)

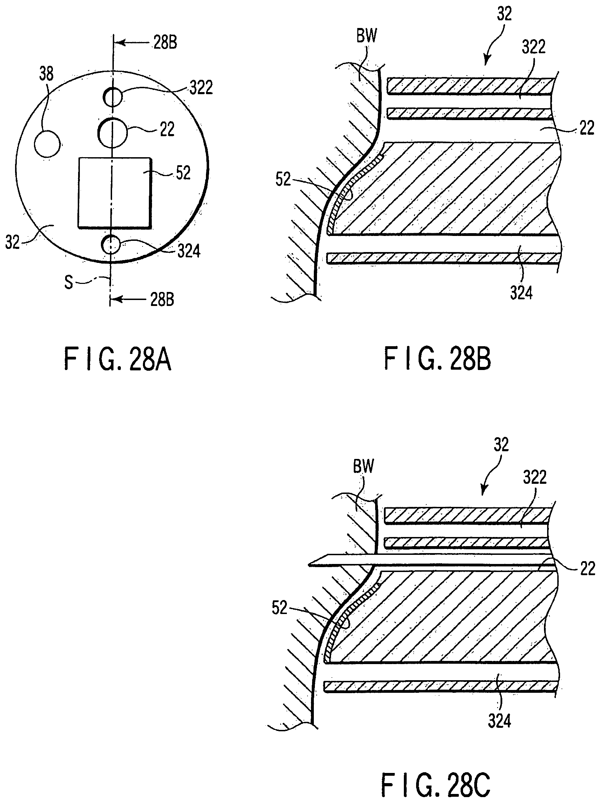

A twelfth embodiment, which is a modified example of the eleventh embodiment, will now be described with reference to FIGS. 28A to 28C, in which like numbers indicate like elements and the explanation thereof will be omitted.

As shown in FIGS. 28A and 28B, disposed on the central axis (i.e., oscillating face) S of the ultrasonic transducer 52 are the first suction passage 322, the working channel (forceps channel) 22, and the second suction passage 324. In particular, the first suction passage 322 and the second suction passage 322 are separated. Disposed between the ultrasonic transducer 52 and the first suction passage 322 is the working channel 22.

With this configuration, an area of interest, such as a body cavity wall BW, can be suctioned by the first and second suction passages 322 and 324 and thereby brought into close contact with the distal end face of the insertion section 12 of the ultrasonic endoscope 10. This ensures adequate ultrasonically observed image. In addition, a surgical operation is performed through the working channel 22 while the target area is suctioned so that it is fixed to the distal end of the insertion section 12 of the ultrasonic endoscope 10. Accordingly, surgical operations using the ultrasonic endoscope 10 are facilitated.

In the twelfth embodiment, a description was given exemplifying the case where the working channel 22 is provided independently of the first and second suction passages 322 and 324. However, the first and second suction passages 322 and 324 may together have the function of a working channel.

### (Thirteenth Embodiment)

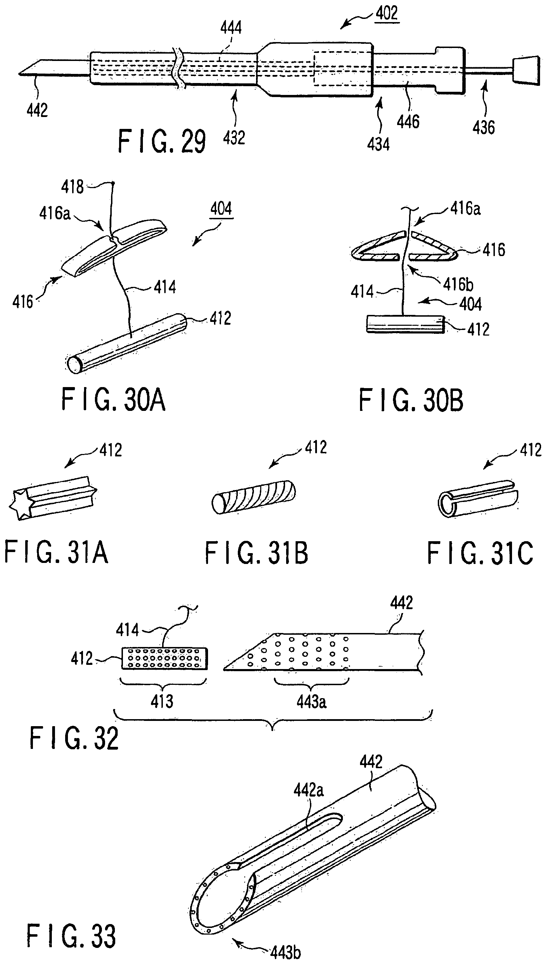

Referring to FIGS. 29 to 39, a thirteenth embodiment will now be described.

An endoscopic system includes the ultrasonic endoscope 10 (see FIG. 1, for example), a T-bar suturing device 402, and a T-bar 404 indwelled using the T-bar suturing device 402.

As shown in FIGS. 30A and 30B, the T-bar 404 includes a bar (or rod) 412, a thread member 414, and a stopper 416. One end of the thread member 414 is fixed to the middle of the bar 412. Disposed on the thread member 414 is the stopper 416 that can be brought into contact with the bar 412 and separated therefrom. At the other end of the thread member 414 is a spherical member 418. The spherical member 418 prevents the stopper 416 from becoming disconnected with the thread member 414. The thread member 414 is made of a material that is curvable by gravity or the like but has a proper degree of stiffness and a proper degree of flexibility such that the material is less likely to be bent by gravity, etc.

As shown in FIG. 30B, the stopper 416 has an approximately isosceles triangular cross-section. Formed at the vertex between the two equal sides of the isosceles triangle is a gripping part 416a that engages with the thread member 414. Formed in the side that is opposite the gripping part 416a is a hole 416b, through which the thread member 414 is passed. The hole 416b of the stopper 416 is closer to the bar 412 than is the gripping part 416a of the stopper 416.

To move the stopper 416 closer to the bar 412 (i.e., to shorten the distance therebetween), the gripping part 416a of the stopper 416 is deformed so as to open. Consequently, the stopper 416 smoothly moves along the thread member 414. To move the stopper 416 away from the bar 412, on the other hand, the gripping part 416a of the stopper 416 is deformed so as to close. As a result, a large frictional force is applied between the stopper 416 and thread member 414 such that the stopper 416 is less likely to move along the thread member 414. Accordingly, the stopper 416 does not move unless a large force is exerted thereon.

In other words, movement of the stopper 416 along the thread member 414 is allowed when the distance between the bar 412 and the stopper 416 is shortened, whereas movement of the stopper 416 along the thread member 414 is restricted when the distance therebetween is lengthened.

In order to distinguish the bar 412 from a needle tube 442 by ultrasonic observation, the bar 412 undergoes various processes in manufacture. As shown in FIGS. 31A to 32, the bar 412 is made different from the needle tube 442, namely in terms of shape and ultrasonic wave reflection. The bar 412 shown in FIG. 31A has an approximately star-shaped cross-section. The bar 412 shown in FIG. 31B has a densely helical form. The bar 412 shown in FIG. 31C has a C-shaped cross-section. The shape of the bar 412 is not limited to the ones shown in FIGS. 31A to 31C, but any shape can be permitted as long as the bar 412 is easily recognizable by making the reflectance of the bar 412 distinct according to ultrasonic waves, thereby causing irregular reflection or the like such that the bar 412 is conspicuous when ultrasonically observed.

As shown in FIG. 32, at least the density, depth, or shape of a dimpled part 443a formed on the side faces of the distal end of the needle tube 442 (described below) is different from those of a dimpled part 413 formed on the bar 412. It is also preferable that the coating on the surface of the bar 412 be different from that on the surface of the needle tube 442. It is further preferable that the material of the bar 412 be clearly distinguishable from that of the needle tube 442 by ultrasonic observation.

As shown in FIG. 33, the edge 443b of the distal end of the needle tube 442 is preferably treated by an ultrasonic-reflection process. In this case, the reflection process of the dimpled part 443a of the periphery of the distal end of the needle tube 442 shown in FIG. 32 preferably differs from that of the edge 443b of the distal end of the needle tube 442 shown in FIG. 33.

Such a reflection process makes ultrasonic wave reflection of the dimpled part 443a of the side faces of the distal end of the needle tube 442, that of the edge 443b of the distal end of the needle tube 442, and that of the bar 412 of the T-bar 404 different from one another. Specifically, this causes different irregular reflections of ultrasonic waves. Accordingly, the edge 443b of the distal end of the needle tube 442, the side faces of the distal end of the needle tube 442, and the bar 412 of the T-bar 404 projecting from the distal end of the needle tube 442 can easily be identified by ultrasonic observation.

Incidentally, as shown in FIG. 29, the T-bar suturing device 402 includes an outer sheath 432, a needle structure 434, and a pusher 436. The needle structure 434 is movable in the cavity of the outer sheath 432. The pusher 436 is movable in the cavity of the needle structure 434. It is necessary that the outer sheath 432 of the T-bar suturing device 402 be insertable in each working channel 22, 24 of the endoscope 10. Therefore, the outside diameter of the outer sheath 432 is slightly smaller than the bore diameter of the working channel 22, 24. The outer sheath 432, needle structure 434, and pusher 436 are longer than the working channels 22 and 24.

The needle structure 434 has the above-mentioned needle tube 442, a flexible tube (inner sheath) 444, and a needle slider 446. Fixed at the distal end of the flexible tube 444 is the needle tube 442, and fixed at the proximal end of the flexible tube 444 is the needle slider 446.

As shown in FIGS. 33 to 35, a slit 442a is formed in the distal end of the needle tube 442. The needle tube 422 has an inside diameter sufficient for the distal end of the bar 412 to be inserted therein. The slit 442a has a width sufficient for the thread member 414 to be disposed therein.

Next, a description will be given of the operation of the endoscopic system according to the thirteenth embodiment.

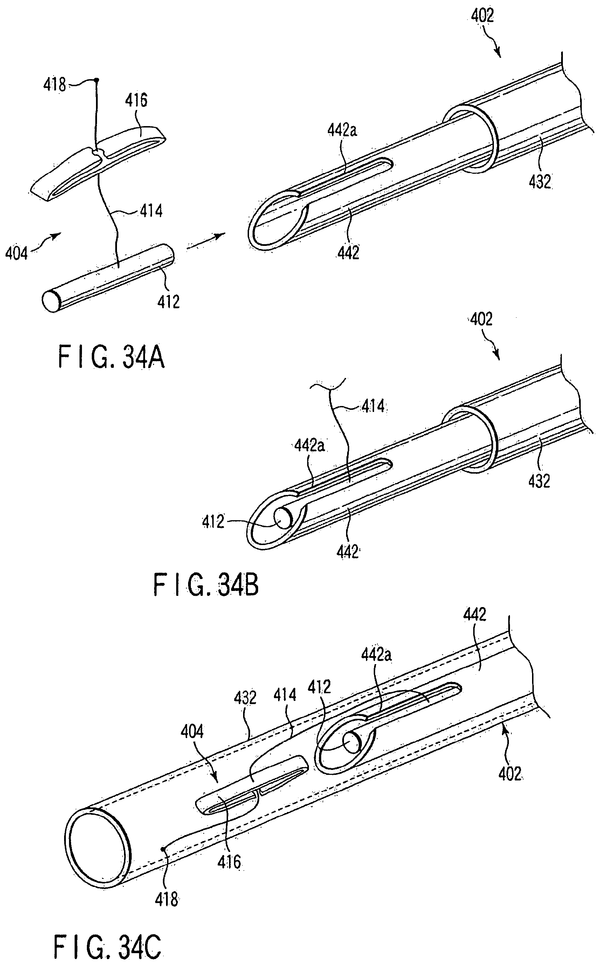

First, the T-bar 404 is attached to the T-bar suturing device 402. As shown in FIG. 34A, the distal end of the needle tube 442 projects from the outer sheath 432. Then, the bar 412 of the T-bar 404 is inserted from the distal end of the needle tube 442. As shown in FIG. 34B, the thread member 414 extends from the needle tube 442 through the slit 442a.

Subsequently, as shown in FIG. 34C, the outer sheath 432 is moved forward in relation to the needle tube 442. As a result, the outer sheath 432 bends the thread member 414 forward. On account of friction, the bar 412 engages with the needle tube 442 and the thread member 414 engages with the internal wall of the outer sheath 432. This prevents the bar 412 from falling off the distal end of the needle tube 442 by mistake. Accordingly, the stopper 416 of the T-bar 404 is kept in the cavity of the distal end of the outer sheath 432.

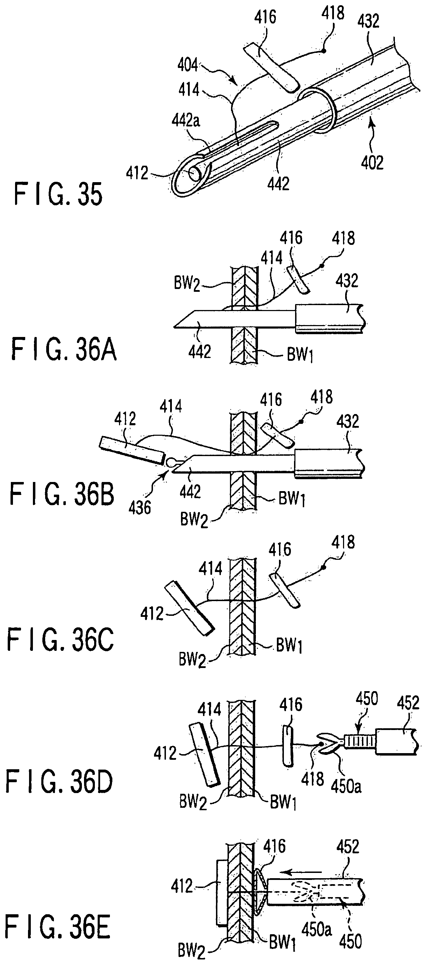

The T-bar suturing device 402 thus prepared is inserted in the first working channel 22. The distal end of the outer sheath 432 of the T-bar suturing device 402 is introduced into a body cavity in an endoscopic manner. Under optical observation, the distal end of the needle tube 442 projects from the outer sheath 432, as shown in FIG. 35. Then, the distal end of the needle tube 442 pierces through the body cavity walls (i.e., the living tissue) BW₁ and BW₂. At this time, the stopper 416 of the T-bar 404 is within the body cavity.

Subsequently, the bar 412 is pushed by the pusher 436, thereby causing the bar 412 to fall from the distal end of the needle tube 442. At this time, the edge 443b of the distal end of the needle tube 442 (see FIG. 33) is recognized by ultrasonic observation. The bar 412 is also recognized when projecting from the distal end of the needle tube 442 (see FIGS. 31A to 31C). Accordingly, whether the bar 412 has fallen from the distal end of the needle tube 442 can be securely determined by ultrasonic observation. At this time, the bar 412 is disposed outside the body cavity, and the stopper 416 is disposed within the body cavity.

Subsequently, a grasping forceps 450 covered with a sheath 452 is inserted in, for example, the second working channel 24, and the thread member 414 or spherical member 418 is grasped with a grasping part 450a of the grasping forceps 450. While the thread member 414 or spherical member 418 is grasped with the grasping part 450a, the sheath 452 is moved in the direction of the front end of the grasping part 450a. Consequently, the tip of the sheath 452 presses the stopper 416 of the T-bar 404 such that the stopper 416 moves closer to the bar 412 along the thread member 414. At this point, the grasping part 450a is released from the grasping state. Accordingly, the lumen walls BW₁ and BW₂ are kept sandwiched between the bar 412 and stopper 416.

The following benefits and advantages are obtained from the thirteenth embodiment.

The bar 412 of the T-bar 404, the edge of the distal end of the needle tube 442, and the side faces of the distal end of the needle tube 442 are made different in the density of the reflection process and shape such that one can easily be distinguished from the others when ultrasonically viewed. Further, they are covered with coatings that enable one to be easily distinguished from the others when ultrasonically viewed. This makes it easy to recognize by ultrasonic observation whether the bar 412 of the T-bar 404 has been caused to fall from the distal end of the needle tube 442.

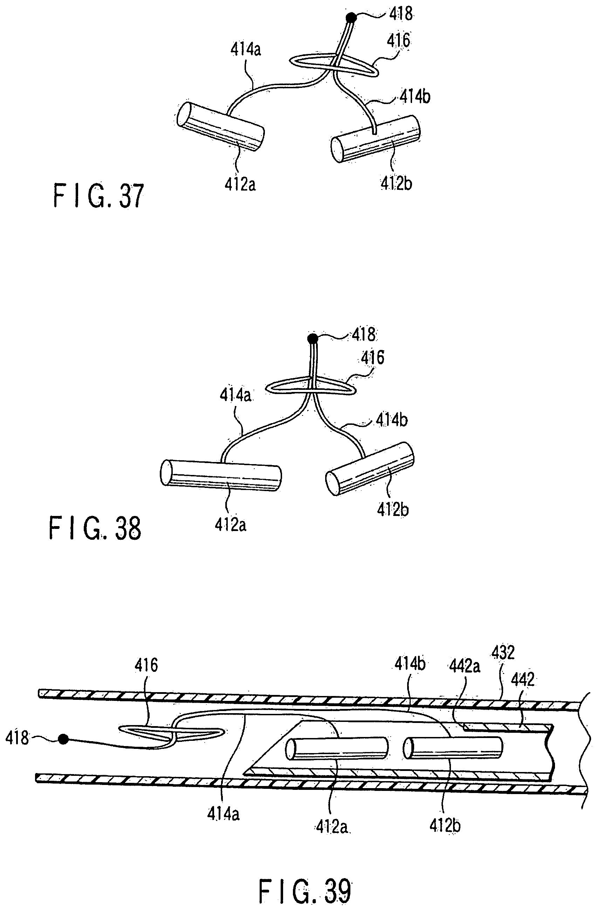

The T-bar 404 varies in type depending on the number of bars 412, for example, a single type (see FIGS. 30A and 30B) that has one bar, as in the foregoing description, and a double type (see FIGS. 37 and 38) that has two bars. It is preferable that bars 412a and 412b of the double type T-bar shown in each of FIGS. 37 and 38 be reflection-processed differently. Needless to say, the bars 412a and 412b are reflection-processed so as to be distinguished from the needle tube 442. The lengths of the bars 412a and 412b of the double type T-bar shown in FIG. 38 are different. Accordingly, when the double type T-bar 404 having the bars 412a and 412b is placed in a body cavity, not only a difference in reflection process between the bars but also a difference in length between them makes it possible to distinguish one from the other.

As shown in FIG. 39, the double type T-bar 404 may have thread members 414a and 414b of different lengths. Specifically, the thread member 414a, connected to one bar 412 disposed on the distal end side of the needle tube 442, is shorter than the thread member 414b connected to the other bar 412 disposed behind the one bar. Accordingly, the T-bars 404 can be satisfactorily loaded into the T-bar suturing device 402. In addition, the T-bars 404 can also be easily loaded into the needle tube 422 and outer sheath 432 of the T-bar suturing device 402.

### (Fourteenth Embodiment)

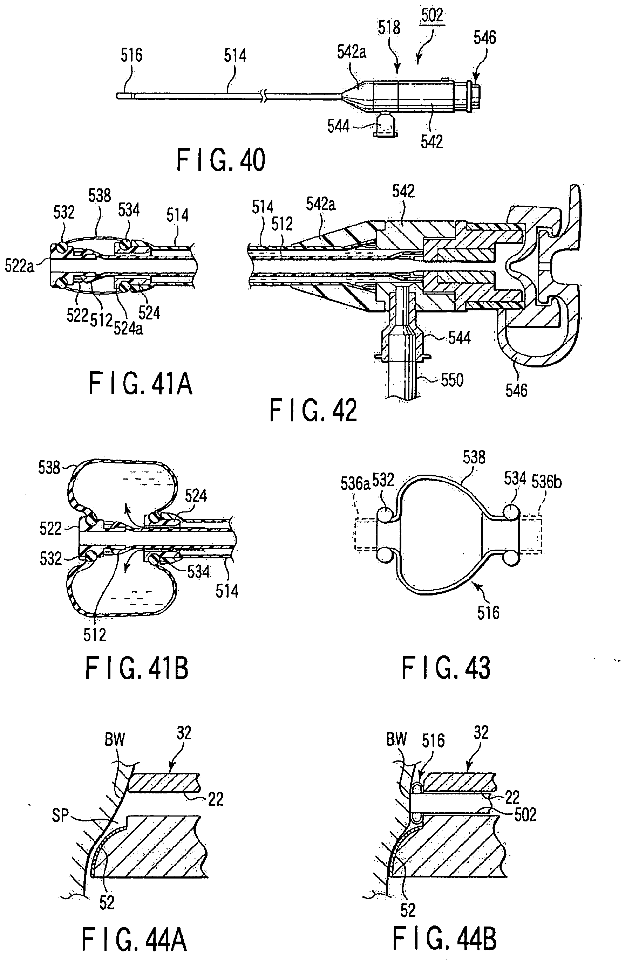

A fourteenth embodiment, which is a modified example of the thirteenth embodiment, will now be described with reference to FIGS. 40 to 44B, in which like numbers indicate like elements and the explanation thereof will be omitted.

A sheath 502 shown in FIG. 40 is inserted in the working channel 22 or 24 of the ultrasonic endoscope 10. As shown in FIGS. 41A to 43, the sheath 502 includes an inner sheath 512, an outer sheath 514, a balloon 516, and a holding portion 518.

Examples of the sheath having such a balloon 516 include the outer sheath 432 (see FIG. 34C) of the T-bar suturing device 402 used to place the T-bar 404 described in the thirteenth embodiment. That is, the balloon 516 is preferably attached to the distal end of such a sheath 432. Now, a description is given exemplifying the case where the sheath according to the fourteenth embodiment, represented by reference numeral 502, is used as the sheath 432 described in the thirteenth embodiment.

As shown in FIGS. 41A to 42, the outer sheath 514 is on the outside of the inner sheath 512. The distal end of the inner sheath 512 projects beyond the distal end of the outer sheath 514, as shown in FIGS. 41A and 41B. A first mouthpiece 522 is fitted to the distal end of the inner sheath 512. Formed in the periphery of the first mouthpiece 522 is a first recess 522a, in which a distal end side O-ring 532 (described below) of the balloon 516 fits. Likewise, a second mouthpiece 524 is fitted to the distal end of the outer sheath 514. Formed in the periphery of the second mouthpiece 524 is a second recess 524a, in which a proximal end side O-ring 534 (described below) of the balloon 516 fits. Disposed between the first and second mouthpieces 522 and 524 is the balloon 516.

The balloon 516 includes the distal end side O-ring 532, the proximal end side O-ring 534, thin-diameter parts 536a and 536b, and an inflatable part 538. The thin diameter parts 536a and 536b are formed on the distal end side and proximal end side, respectively, of the inflatable part 538. The distal end side O-ring 532 and the proximal end side O-ring 534 are disposed on the thin diameter parts 536a and 536b, respectively. Disposed on the thin diameter parts 536a and 536b are the distal end side O-ring 532 and proximal end side O-ring 534 fitted in the recess 522a of the first mouthpiece 522 and the recess 524b of the second mouthpiece 524, respectively. The inflatable part 538 is symmetrical around its longitudinal axis, as shown in FIG. 43. Additionally, the inflatable part 538 is formed asymmetrically such that the outside diameter once increases from the distal end side toward the proximal end side and then gradually decreases. To be specific, the tangents touching the periphery of the inflatable part 538 and parallel to the longitudinal axis of the balloon 516 are located closer to the distal end side O-ring 532 than to the proximal end side O-ring 534. Accordingly, the balloon 516 is inflated outward in the direction of the distal end of the balloon 516, as shown in FIG. 41B.

Incidentally, with the balloon 516 kept between the first and second mouthpieces 522 and 524, the distance between the first and second recesses 522a and 524a of the first and second mouthpieces 522 and 524 is properly set in order to prevent the outside diameter of the inflatable part 538 from becoming greater than the inside diameter of the working channel 22 and thus making it difficult for the inflatable part to be inserted in the working channel 22. Specifically, the diameter of the inflatable part 538 is kept as small as possible while the inflatable part 538 is longitudinally extended.

Next, as shown in FIG. 42, the holding portion 518 includes a fixing part 542 having a bend preventing portion 542a, a syringe connector 544, and a surgical instrument holding plug 546. The fixing part 542 fixes the proximal ends of the inner and outer sheathes 512 and 514. The syringe connector 544 is attached to the fixing part 542. A detachable syringe 550 can be attached to the syringe connector 544. Using the syringe 550, a liquid can be injected or discharged between the outer face of the inner sheath 512 and the inner face of the outer sheath 514. Disposed at the proximal end of the fixing part 542 is the surgical instrument holding plug 546, which is made of, for example, a rubber material. The surgical instrument holding plug 546 holds a surgical instrument inserted in the inner sheath 512, and also prevents any fluid or the like in the body cavity from being discharged through the inner sheath 512.

Next, a description is given of an operation exemplifying the case where the sheath 502 according to the fourteenth embodiment is disposed in the working channel 22 of the ultrasonic endoscope 10 and used.

As shown in FIG. 44A, when the ultrasonic transducer 52 at the distal end of the insertion section 12 of the ultrasonic endoscope 10 is brought into contact with a body wall, a space is defined between the ultrasonic transducer 52 and the body wall. As a result, an ultrasonically observed image to check whether the bar 412 of the T-bar 404 is projecting from the distal end of the needle tube 422 of the T-bar suturing device 402 may be only partial.

In such a case, the sheath 502 having the balloon 516 at its tip is used instead of the outer sheath 432 of the T-bar suturing device 402, as shown in FIG. 44B. The sheath 502 of the T-bar suturing device 402 having the balloon 516 at the tip is projected from the distal end of the working channel 22. Subsequently, while projected from the distal end of the insertion section 12 of the ultrasonic endoscope 10, the balloon 516 is inflated by the syringe. Consequently, as shown in FIG. 41B, the balloon 516 is inflated beyond the distal end of the first mouthpiece 522 at the tip of the inner sheath 512, and also inflated radially outward. As a result, the balloon 516 comes into contact with a body wall as well as the ultrasonic transducer 52. Thus, the oscillation of the ultrasonic transducer 52 is transmitted to the body wall via the balloon 516. Since the space between the ultrasonic transducer 52 and the body wall is filled by the balloon 516, a more accurate ultrasonically observed image can be obtained.

At this point, insertion in the first working channel 22 takes place. The tip of the sheath 502 of the T-bar suturing device 402 is introduced into a body cavity in an endoscopic manner. Then, under optical observation, the distal end of the needle tube 442 projects from the sheath 502, and pierces through the body cavity walls (the tissues of a body cavity) BW₁ and BW₂. The syringe 550 is attached to the holding portion 518 and the balloon 516 is inflated with a liquid frontward and radially outward. Then, for example, by holding the holding portion 518, the sheath 502 is pulled toward the proximal end and the balloon 516 is hooked around the edge of the open end 22a of the working channel 22. At this time, an adjustment is made so that the distal end of the needle tube 442 does not move.

This condition is ultrasonically observed. Consequently, as shown in FIG. 44B, the balloon fills the space. Accordingly, voids in the ultrasonically observed image can be minimized.

Subsequently, the bar 412 is pressed with the pusher 436 and caused to fall from the distal end of the needle tube 442. At this time, the edges 443b (see FIG. 33) of the distal end of the needle tube 442 can be recognized by ultrasonic observation. The bar 412 (see FIGS. 31A to 31C) projecting from the distal end of the needle tube 442 can also be recognized by ultrasonic observation. Accordingly, it is securely determined through ultrasonic observation whether the bar 412 has fallen from the distal end of the needle tube 442. At this time, the bar 412 is disposed outside the body cavity, and the stopper 416 inside the body cavity.

The following benefits and advantages are obtained from the fourteenth embodiment.

Injecting the liquid in the balloon 516 of the sheath 502 inflates the balloon 516 forward and radially outward. This enables the balloon 516 to be disposed on the distal end face of the insertion section 12 of the endoscope 10 so as to be adjacent to the ultrasonic transducer 52. Consequently, the balloon 516 filled with a medium that transmits ultrasonic oscillation comes into close contact with the tissues of a body cavity. Accordingly, a satisfactory ultrasonically observed image can be obtained.

In the fourteenth embodiment, a description was given exemplifying the case where the sheath 502 having the balloon 516 at the tip is provided instead of the outer sheath 432 of the T-bar suturing device 402 described in the thirteenth embodiment. However, it is also preferable that the T-bar suturing device 402 described in the thirteenth embodiment be inserted in the cavity of the sheath 502 in the fourteenth embodiment in order to perform surgical operations.
Additional advantages and modifications will readily occur to those skilled in the art. Therefore, the invention in its broader aspects is not limited to the specific details and representative embodiments shown and described herein. Accordingly, various modifications may be made.

## Claims

1. An ultrasonic endoscope (10) comprising:
an insertion section (12) having a distal end and a proximal end;
an operation section (14) disposed on the proximal end of the insertion section (12);
an optical observation system disposed in the insertion section (12), the optical observation system having an objective lens (38) on a distal end surface of the insertion section (12);
an ultrasonic observation system disposed in the insertion section (12), the ultrasonic observation system having an ultrasonic transducer (52; 52a; 52c) on the distal end surface of the insertion section (12) or further forward than the distal end thereof; and
at least two working channels (22, 24) provided in the insertion section (12),
**characterized in that** the ultrasonic observation system includes:
an ultrasonic probe channel (26) having a distal end and disposed in the insertion section (12); and
an ultrasonic probe (50) having a distal end at which the ultrasonic transducer (52; 52a; 52c) is disposed, the ultrasonic probe (50) being inserted in the probe channel (26) so as to be rotatable around its axis, wherein the ultrasonic probe (50) is rotatable when the distal end of the ultrasonic probe (50) is projected further forward than the distal end of the insertion section (12), and the distal end of the ultrasonic probe (50) is fixable to the distal end of the insertion section (12) when pulled inside the distal end of the insertion section (12) such that a central axis of the ultrasonic transducer (52; 52a; 52c) is directed to at least one of the two working channels (22, 24), and
the optical observation system is disposed at the distal end of the insertion section (12) so as to be substantially equidistant from at least the two working channels (22, 24).

2. The ultrasonic endoscope (10) according to claim 1, **characterized in that** the optical observation system is disposed between the two working channels (22, 24).

3. The ultrasonic endoscope (10) according to claim 1, **characterized in that** the ultrasonic transducer (52; 52a; 52c) is disposed on a tip of the distal end of the ultrasonic probe (50).

4. The ultrasonic endoscope (10) according to claim 3, **characterized in that** the ultrasonic transducer (52b; 52c) is disposed on a side face of the distal end of the ultrasonic probe (50).

5. The ultrasonic endoscope (10) according to claim 1, **characterized in that**
at least one first fixing part (60) is disposed at the distal end of the ultrasonic probe (50), and
the ultrasonic probe channel (26) is provided with a plurality of second fixing parts (26d, 26e) that engage with the first fixing part (60).

6. The ultrasonic endoscope (10) according to claim 5, **characterized in that**
the first fixing part (60) is provided with at least one projection that projects away from the axis of the ultrasonic probe (50), and
the second fixing part (26d, 26e) is provided with a plurality of recesses that engage with the projection.

7. The ultrasonic endoscope (10) according to claim 1, **characterized in that**
a periphery of the distal end of the ultrasonic probe (50) has a shape of an approximately regular polygon,
edges of the distal end of the probe channel (26) form an approximately regular polygon to which the distal end of the ultrasonic probe (50) fits, and
the working channel (22, 24) is disposed on a line passing through the center of the ultrasonic probe channel (26) and perpendicular to the edge of the ultrasonic probe channel (26).

8. The ultrasonic endoscope (10) according to claim 7, **characterized in that**
the distal end of the ultrasonic probe (50) has a shape of an approximately regular polygonal prism, and
the distal end of the probe channel (26) has a shape of an approximately regular polygon.

9. The ultrasonic endoscope (10) according to claim 1, **characterized in that**
at least one of the two working channels (22, 24) has an insertion axis (α, D) that intersects a scanning surface (S) of the ultrasonic transducer (52; 52a; 52c).

10. The ultrasonic endoscope (10) according to claim 1, **characterized in that**
at least one of the two working channels (22, 24) has an inclined axis (α, D) to a lengthwise direction of the insertion section (12), and
the ultrasonic transducer (52; 52a; 52c) has a scanning surface (S) of the ultrasonic transducer (52; 52a; 52c) that intersects the central axis of the surgical instrument on the inclined axis when a surgical instrument is inserted in the working channel (22, 24) having the inclined axis.

11. The ultrasonic endoscope (10) according to claim 1, **characterized in that**
the ultrasonic transducer (52a; 52b; 52c) is disposed on the distal end surface and one side face of the ultrasonic probe (50).

12. The ultrasonic endoscope (10) according to claim 1, **characterized in that** the working channel (22, 24) has a central axis that intersects a scanning surface (S) of the ultrasonic transducer (52; 52a; 52c).

## Patentansprüche

1. Ultraschallendoskop (10) umfassend:
einen Einführungsabschnitt (12), der ein distales Ende und ein proximales Ende aufweist;
einen Operationsabschnitt (14), der an dem proximalen Ende des Einführungsabschnitts (12) angeordnet ist;
ein optisches Beobachtungssystem, das in dem Einführungsabschnitt (12) angeordnet ist, wobei das optische Beobachtungssystem eine Objektivlinse (38) an einer distalen Endseite des Einführungsabschnitts (12) aufweist;
ein Ultraschallbeobachtungssystem, das in dem Einführungsabschnitt (12) angeordnet ist, wobei das Ultraschallbeobachtungssystem einen Ultraschallwandler (52; 52a; 52c) auf der distalen Endseite des Einführungsabschnitts (12) oder weiter vorne als das distalen Ende desselben aufweist; und
mindestens zwei Arbeitskanäle (22, 24), die in dem Einführungsabschnitt (12) bereitgestellt sind,
**dadurch gekennzeichnet, dass** das Ultraschallbeobachtungssystem umfasst:
einen Ultraschallsondenkanal (26), der ein distales Ende aufweist und in dem Einführungsabschnitt (12) angeordnet ist; und
eine Ultraschallsonde (50), die ein distales Ende aufweist, an welchem der Ultraschallwandler (52; 52a; 52c) angeordnet ist, wobei die Ultraschallsonde (50) so in den Sondenkanal (26) eingeführt ist, um um ihre Achse drehbar zu sein,
wobei die Ultraschallsonde (50) drehbar ist, wenn das distale Ende der Ultraschallsonde (50) weiter hervorsteht als das distale Ende des Einführungsabschnitts (12), und das distale Ende der Ultraschallsonde (50) an dem distalen Ende des Einführungsabschnitts (12) fixierbar ist, wenn es in das distale Ende des Einführungsabschnitt (12) so hineingezogen wird, dass eine zentrale Achse des Ultraschallwandlers (52; 52a; 52c) auf mindestens einen der beiden Arbeitskanäle (22, 24) gerichtet ist, und
das optische Beobachtungssystem an dem distalen Ende des Einführungsabschnitts (12) so angeordnet ist, um im Wesentlichen äquidistant von den mindestens zwei Arbeitskanälen (22, 24) zu sein.

2. Ultraschallendoskop (10) gemäß Anspruch 1, **dadurch gekennzeichnet, dass**
das optische Beobachtungssystem zwischen den beiden Arbeitskanälen (22, 24) angeordnet ist.

3. Ultraschallendoskop (10) gemäß Anspruch 1, **dadurch gekennzeichnet, dass** der Ultraschallwandler (52; 52a; 52c) an einer Spitze des distalen Endes der Ultraschallsonde (50) angeordnet ist.

4. Ultraschallendoskop (10) gemäß Anspruch 3, **dadurch gekennzeichnet, dass** der Ultraschallwandler (52b; 52c) an einer Seitenfläche des distalen Endes der Ultraschallsonde (50) angeordnet ist.

5. Ultraschallendoskop (10) gemäß Anspruch 1, **dadurch gekennzeichnet, dass**
mindestens ein erstes Befestigungsteil (60) an dem distalen Ende der Ultraschallsonde (50) angeordnet ist, und
der Ultraschallsondenkanal (26) mit einer Vielzahl von zweiten Befestigungsteilen (26d, 26e) versehen ist, welche mit dem ersten Befestigungsteil (60) in Eingriff treten.

6. Ultraschallendoskop (10) gemäß Anspruch 5, **dadurch gekennzeichnet, dass**
das erste Befestigungsteil (60) mit mindestens einem Vorsprung versehen ist, welcher von der Achse der Ultraschallsonde (50) hervorsteht, und
das zweite Befestigungsteil (26d, 26e) mit einer Mehrzahl von Vertiefungen versehen ist, welche mit dem Vorsprung in Eingriff treten.

7. Ultraschallendoskop (10) gemäß Anspruch 1, **dadurch gekennzeichnet, dass**
eine Umgebung des distalen Endes der Ultraschallsonde (50) eine Form eines ungefähr regelmäßigen Polygons aufweist,
Kanten des distalen Endes des Sondenkanals (26) ein ungefähr regelmäßiges Polygon ausbilden, zu welchem das distale Ende der Ultraschallsonde (50) passt, und
der Arbeitskanal (22, 24) auf einer Linie angeordnet ist, welche durch das Zentrum des Ultraschallsondenkanals (26) und senkrecht zu der Kante des Ultraschallsondenkanals (26) verläuft.

8. Ultraschallendoskop (10) gemäß Anspruch 7, **dadurch gekennzeichnet, dass**
das distale Ende der Ultraschallsonde (50) eine Form eines ungefähr regelmäßigen Polygonprismas aufweist, und
das distale Ende des Sondenkanals (26) eine Form eines ungefähr regelmäßigen Polygons aufweist.

9. Ultraschallendoskop (10) gemäß Anspruch 1, **dadurch gekennzeichnet, dass**
mindestens einer der beiden Arbeitskanäle (22, 24) eine Einführungsachse (α, D) aufweist, welche eine Abtastoberfläche (S) des Ultraschallwandlers (52; 52a; 52c) schneidet.

10. Ultraschallendoskop (10) gemäß Anspruch 1, **dadurch gekennzeichnet, dass**
mindestens einer der beiden Arbeitskanäle (22, 24) eine geneigte Achse (α, D) zu einer Längsrichtung des Einführungsabschnitt (12) aufweist, und
der Ultraschallwandler (52; 52a; 52c) eine Abtastoberfläche (S) des Ultraschallwandlers (52; 52a; 52c) aufweist, welche die zentrale Achse des chirurgischen Instruments auf der geneigten Achse schneidet, wenn ein chirurgisches Instrument in den Arbeitskanal (22, 24) eingeführt wird, welcher die geneigte Achse aufweist.

11. Ultraschallendoskop (10) gemäß Anspruch 1, **dadurch gekennzeichnet, dass**
der Ultraschallwandler (52a; 52b; 52c) auf der distalen Endseite und einer Seitenfläche der Ultraschallsonde (50) angeordnet ist.

12. Ultraschallendoskop (10) gemäß Anspruch 1, **dadurch gekennzeichnet, dass** der Arbeitskanal (22, 24) eine zentrale Achse aufweist, welche eine Abtastoberfläche (S) des Ultraschallwandlers (52; 52a; 52c) schneidet.

## Revendications

1. Endoscope ultrasonore (10) comprenant :
une section d'insertion (12) comportant une extrémité distale et une extrémité proximale ;
une section d'actionnement (14) disposée sur l'extrémité proximale de la section d'insertion (12) ;
un système d'observation optique disposé dans la section d'insertion (12), le système d'observation optique comportant une lentille d'objectif (38) sur une surface d'extrémité distale de la section d'insertion (12) ;
un système d'observation ultrasonore disposé dans la section d'insertion (12), le système d'observation ultrasonore comportant un transducteur ultrasonore (52 ; 52a ; 52c) sur la surface d'extrémité distale de la section d'insertion (12) ou davantage vers l'avant par rapport à l'extrémité distale de celle-ci, et
au moins deux canaux de travail (22, 24) prévus dans la section d'insertion (12),
**caractérisé en ce que** le système d'observation ultrasonore comprend :
un canal de sonde ultrasonore (26) comportant une extrémité distale et disposé dans la section d'insertion (12) ; et
une sonde ultrasonore (50) comportant une extrémité distale au niveau de laquelle le transducteur ultrasonore (52) est disposé, la sonde ultrasonore (50) étant insérée dans le canal de sonde (26) de façon à être rotative autour de son axe,
dans lequel la sonde ultrasonore (50) est rotative lorsque l'extrémité distale de la sonde ultrasonore (50) fait saillie davantage vers l'avant par rapport à l'extrémité distale de la section d'insertion (12), et l'extrémité distale de la sonde ultrasonore (50) peut être fixée à l'extrémité distale de la section d'insertion (12) lorsqu'elle est tirée à l'intérieur de l'extrémité distale de la section d'insertion (12) d'une manière telle qu'un axe central du transducteur ultrasonore (52 ; 52a ; 52c) est dirigé vers au moins l'un des deux canaux de travail (22, 24), et
le système d'observation optique est disposé au niveau de l'extrémité distale de la section d'insertion (12) de façon à être sensiblement équidistant d'au moins les deux canaux de travail (22, 24).

2. Endoscope ultrasonore (10) selon la revendication 1, **caractérisé en ce que**
le système d'observation optique est disposé entre les deux canaux de travail (22, 24).

3. Endoscope ultrasonore (10) selon la revendication 1, **caractérisé en ce que** le transducteur ultrasonore (52 ; 52a ; 52c) est disposé sur une pointe de l'extrémité distale de la sonde ultrasonore (50).

4. Endoscope ultrasonore (10) selon la revendication 3, **caractérisé en ce que** le transducteur ultrasonore (52b ; 52c) est disposé sur une face latérale de l'extrémité distale de la sonde ultrasonore (50).

5. Endoscope ultrasonore (10) selon la revendication 1, **caractérisé en ce que**
au moins une première partie de fixation (60) est disposée au niveau de l'extrémité distale de la sonde ultrasonore (50), et
le canal de sonde ultrasonore (26) est muni d'une pluralité de deuxièmes parties de fixation (26d, 26e) qui s'engagent avec la première partie de fixation (60).

6. Endoscope ultrasonore (10) selon la revendication 5, **caractérisé en ce que**
la première partie de fixation (60) est munie d'au moins une saillie qui fait saillie en s'éloignant de l'axe de la sonde ultrasonore (50), et
la deuxième partie de fixation (26d, 26e) est munie d'une pluralité d'évidements qui s'engagent avec la saillie.

7. Endoscope ultrasonore (10) selon la revendication 1, **caractérisé en ce que**
une périphérie de l'extrémité distale de la sonde ultrasonore (50) présente la forme d'un polygone approximativement régulier,
les bords de l'extrémité distale du canal de sonde (26) forment un polygone approximativement régulier sur lequel s'ajuste l'extrémité distale de la sonde ultrasonore (50), et
le canal de travail (22, 24) est disposé sur une ligne traversant le centre du canal de sonde ultrasonore (26) et perpendiculaire au bord du canal de sonde ultrasonore (26).

8. Endoscope ultrasonore (10) selon la revendication 7, **caractérisé en ce que**
l'extrémité distale de la sonde ultrasonore (50) présente la forme d'un prisme polygonal approximativement régulier, et
l'extrémité distale du canal de sonde (26) présente la forme d'un polygone approximativement régulier.

9. Endoscope ultrasonore (10) selon la revendication 1, **caractérisé en ce que**
au moins l'un des deux canaux de travail (22, 24) présente un axe d'insertion (a, D) qui croise une surface de balayage (S) du transducteur ultrasonore (52 ; 52a ; 52c).

10. Endoscope ultrasonore (10) selon la revendication 1, **caractérisé en ce que**
au moins l'un des deux canaux de travail (22, 24) présente un axe incliné (a, D) dans le sens de la longueur de la section d'insertion (12), et
le transducteur ultrasonore (52 ; 52a ; 52c) présente une surface de balayage (S) du transducteur ultrasonore (52 ; 52a ; 52c) qui croise un axe central de l'instrument chirurgical sur l'axe incliné lorsqu'un instrument chirurgical est inséré dans le canal de travail (22, 24) possédant l'axe incliné.

11. Endoscope ultrasonore (10) selon la revendication 1, **caractérisé en ce que**
le transducteur ultrasonore (52 ; 52b ; 52c) est disposé sur la surface d'extrémité distale et une face latérale de la sonde ultrasonore (50).

12. Endoscope ultrasonore (10) selon la revendication 1, **caractérisé en ce que** le canal de travail (22, 24) comporte un axe central qui croise une surface de balayage (S) du transducteur ultrasonore (52 ; 52a ; 52c).
